# EUROPEAN PATENT APPLICATION

(11) **EP 2 230 014 A1**
(43) Date of publication of application: **22.09.2010**
(21) Application number: 09250796.1
(22) Date of filing: 20.03.2009
(51) Int. Cl.: B01L 3/00

(54) **Devices and methods for production of cell aggregates**

(71) Applicant: Ungrin, Mark, Oakville, ON L6M 3J9 (CA); Zandstra, Peter, Toronto, ON M4R 1Y2 (CA)
(72) Inventor: Ungrin, Mark, Oakville, ON L6M 3J9 (CA); Zandstra, Peter, Toronto, ON M4R 1Y2 (CA)
(74) Representative: Wright, Andrew John

(57) **Abstract**

A microwell device comprises a fluid vessel having a bottom region. A microwell plate is at the bottom region. The microwell plate comprises an upper region defining an upper plane. A plurality of microwells extend downwardly from the upper plane. Each of the microwells comprisesan axis extending perpendicularly to the upper plane. Each of the microwells comprises a sidewall. The sidewall of at least one of the microwells has at least one wall component extending inwardly towards the axis.

## Description

### FIELD

The present application relates to devices and methods for the formation of cell aggregates, preferably of pluripotent stem cells such as embryonic stem cells. Such cell aggregates are used for the differentiation of pluripotent stem cells such as embryonic stem cells, in the fields of developmental biology, cellular therapies and regenerative medicine.

### INTRODUCTION

The following is not an admission that anything discussed below is prior art or part of the common general knowledge of persons skilled in the art.

Early embryogenesis is a complex but highly organized process. Specific genetic programs, activated in response to positional and intracellular cues allow the progeny of a single cell to self-organize into tissues, organs, and entire organisms. Human embryonic stem cells (hESC), thought to reflect the pluripotency of the inner cell mass, can be maintained in culture and differentiated into a wide range of cell types. One example of this is the conversion of adherent cultures of hESC to extra-embryonic endoderm via the effects of Bone Morphogenetic Protein-2 (BMP2) (Pera_2004). This is useful in basic research into the processes that control embryogenesis, but also as a potential source of specific cell types for the repair and regeneration of damaged tissues in clinical applications, in the field known as regenerative medicine or tissue engineering.

In order to recapitulate some of the cues inherent to *in vivo* differentiation, many hESC differentiation protocols employ 3-dimensional aggregates known as Embryoid Bodies (EBs) as an intermediate step. While EBs permit the generation of cells arising from all three primary germ layers, when generated from human ESC, they are commonly derived as a heterogeneous mixture by scraping monolayer cultures to release colonies, and EB differentiation is a chaotic and disorganized process. Consequently, the precision of *in vivo* morphogenesis, where every cell has its place, gives way to differentiation at the level of population averages. The results are inefficiency and contamination with residual, potentially tumourigenic stem cells. Furthermore, current techniques of EB formation are labour intensive, and not amenable to the automation and scale-up required to produce clinically useful quantities of differentiated cells.

Two recent publications have begun to address the controlled production of EBs in 96-well "spin-EB" format (Ng_2005;Burridge_2007), however neither publication addresses variables that impact the reproducibility of EB formation (such as the shape and volume of the aggregation wells); neither publication addresses the production of EBs from higher density plate formats or arrays of microwells, nor addresses the practical problem of extracting large numbers of EBs from the wells where they are formed, nor the possibility of employing controlled EB production to generate tissue-level order within EBs. In addition, hESC cultured using standard techniques do not consistently form high-quality aggregates (aggregates are often loose, poorly defined and/or cannot be recovered intact) using these methods.

Two publications address the cultivation of embryonic stem cells [ESC] in micron-scale wells to generate defined EBs colony sizes (Khademhosseini_2006;Mohr_2006), as a precursor to formation of EBs of defined size, however both cases employ vertical sidewalls, widely spaced wells, and require cultivation of hESC within the microwells for at least several days. In one case (Khademhosseini_2006), 95% of cells do not settle into the wells, and in both cases, while EB consistency is improved over standard scraping techniques, substantial non-uniformities still exist. When the neurectodermal differentiation pathway is interfered with via overexpression of the *Nodal* gene product (Vallier_2004), some tissue-level order can be seen in human EBs, however this approach is deficient in that it both interferes with normal differentiation pathways and requires genetic modification of the stem cells.

PCT Patent Application Publication WO 2005/007796 to Molecular Cytomics Ltd. (filed on July 20, 2004) discloses a multi-well plate having picowells of dimensions of less than 200 microns. The picowells are useful for the study of individual cells. The picowells generally comprise a volume defined by vertically extending sidewalls, and a base.

Therefore there is a need in the art for a method to reproducibly and efficiently generate consistent cell aggregates, such as stem cell aggregates or embryoid bodies from mammalian embryonic stem cells. There is also a need in the art for a method of reproducibly and efficiently generating tissue-level organization within cell aggregates of mammalian pluripotent stem cells, such as mammalian embryonic stem cell aggregates or embryoid bodies. There is also a need in the art for a method that produces cell aggregates at higher densities and a method for recovering large numbers of cell aggregates from the device in which they are generated, which may be applied to large-scale production and automation in order to provide clinically useful numbers of differentiated cells. Finally there is a need for a method that efficiently produced aggregates with little loss of cells and in a time frame that is short enough to allow for subsequent experimentation and manipulation.

### SUMMARY

The following summary is provided to introduce the reader to the more detailed discussion to follow. The summary is not intended to limit or define the claims.

The present application relates to devices and methods useful for preparing cell aggregates.

In one embodiment, the device is a microwell device with a high density of microwells with limited spacing between the microwells. The high density forces cells into the wells and not outside on the plate. The device is characterized by wells having at least one non-vertical sidewall. Preferably all of the sidewalls are non-vertical and have a substantially constant slope that converge to a point, such that for a microwell of given dimensions, any number of cells from 2 up to the volumetric capacity of the microwell will, when deposited in the microwell (via gravity or centrifugation), be forced into contact with one another. Therefore, the device is designed such that a broad continuum of aggregate sizes can be generated from microwells of a single size, with the aggregate size depending only on the number of cells deposited into each microwell.

Accordingly, in one embodiment, the present invention provides a microwell device comprising:
a) a body comprising an upper region defining an upper plane;
b) a plurality of wells extending downwardly from the upper plane into the body;
c) each of the wells comprising an axis extending perpendicularly to the upper plane; and
d) each of the wells comprising a sidewall, the sidewall of at least one of the wells having at least one wall component extending inwardly towards the axis.

Preferably, at least one wall component is at an angle of less than 90° with respect to the upper plane, more preferably the angle is between 20° and 80°, most preferably between 50° and 60°.

The present application includes the use of the microwell device to prepare cell aggregates and methods for preparing cell aggregates on the device.

The present application further relates to a method for extracting cell aggregates from well plates via centrifugation of an assembled device. The present application also relates to a device that may be assembled for extracting cell aggregates from well plates via centrifugation of the assembled device.

In one embodiment, the application also provides a method of recovering cell aggregates from wells of a source plate using inverted centrifugation or "spin-out" technology. The method involving centrifugation of an assembled device, wherein the source plate is inverted; and wherein the assembled device comprises:
a) the source plate; and
b) a single unit further comprising an alignment collar attached to a collecting plate;
wherein the alignment collar is attached perpendicularly to a base of the collecting plate; and
wherein the alignment collar of the single unit fits outside the perimeter of the source plate and aligns the source plate so that the source plate fits inside the alignment collar when the source plate and single unit of the device are assembled; and
wherein the cell aggregates from the wells of the source plate are collected into the collecting plate during centrifugation.

In another embodiment, the cell aggregates are recovered from wells of a source plate using inverted centrifugation or "spin-out" technology comprising a method involving centrifugation of an assembled device, wherein the source plate is inverted; and wherein the assembled device comprises:
a) the source plate;
b) a collecting plate; and
c) a separate alignment collar;
wherein the alignment collar forms a bridge between the collecting plate and source plate; and
wherein the alignment collar fits outside the perimeter of the collecting plate and aligns the source plate, so that the source plate fits inside the alignment collar when the source plate, the collecting plate and the alignment collar of the device are assembled; and
wherein the cell aggregates from the wells of the source plate are collected into the corresponding wells of the collecting plate during centrifugation.

The "spin-out" technology described above for extracting cells or cell aggregates from, for example, mammalian pluripotent stem cells, such as human embryonic stem cells aggregates or EBs from human embryonic stem cells, from standard format well plates permits the use of higher plate densities, and is compatible with the robotics, automation and scale-up that will be required to produce clinically useful numbers of differentiated cells via mammalian pluripotent stem cell aggregates, such as mammalian embryonic stem cell aggregates or EB intermediates.

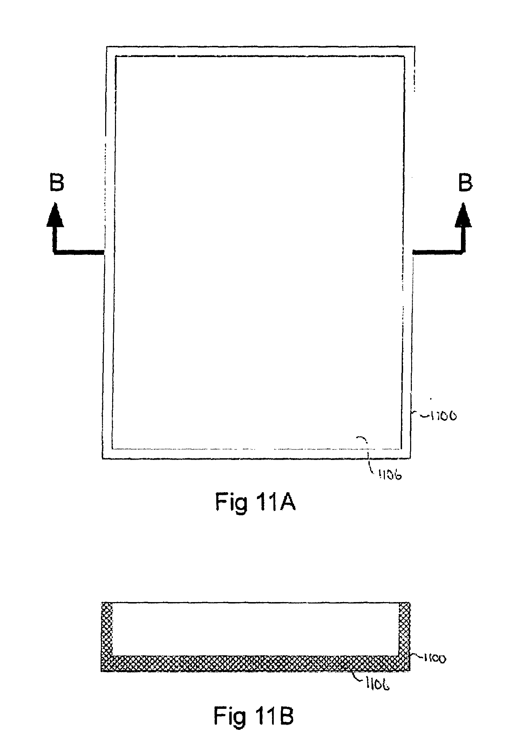

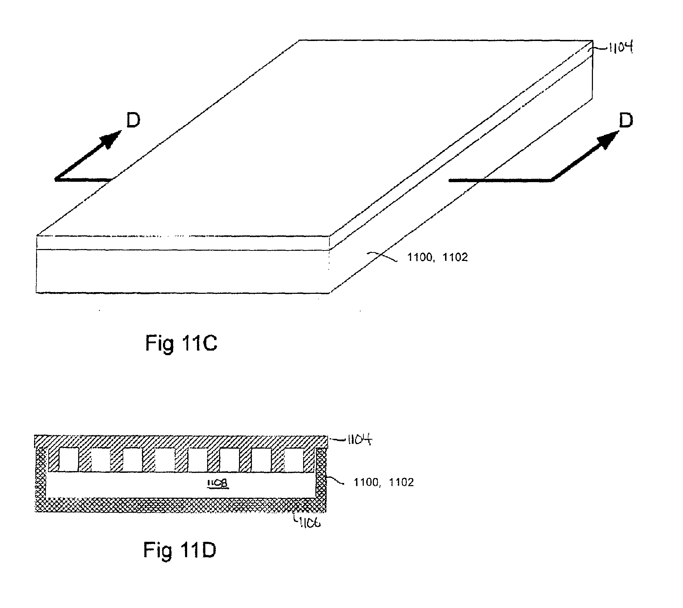

The application also provides a device for recovering cells comprising an alignment collar and integral collecting plate as illustrated in Figure 11. Accordingly, the application discloses a device for recovering cells from wells of a source plate comprising:
a) the source plate; and
b) a single unit further comprising an alignment collar attached to a collecting plate;
wherein the alignment collar is attached perpendicularly to a base of the collecting plate; and
wherein the alignment collar of the single unit fits outside the perimeter of the source plate and aligns the source plate so that the source plate fits inside the alignment collar when the source plate and single unit of the device are assembled.

In one embodiment, the source plate that fits inside the alignment collar is inverted and the cells from the wells of the source plate are collected into the wells of the collecting plate in the assembled device. In another embodiment, the assembled device is centrifuged. In yet another embodiment, the cells recovered from the assembled device are mammalian pluripotent stem cell aggregates such as mammalian embryonic stem cells aggregates or embryoid bodies. In another embodiment, the mammalian pluripotent stem cells are human.

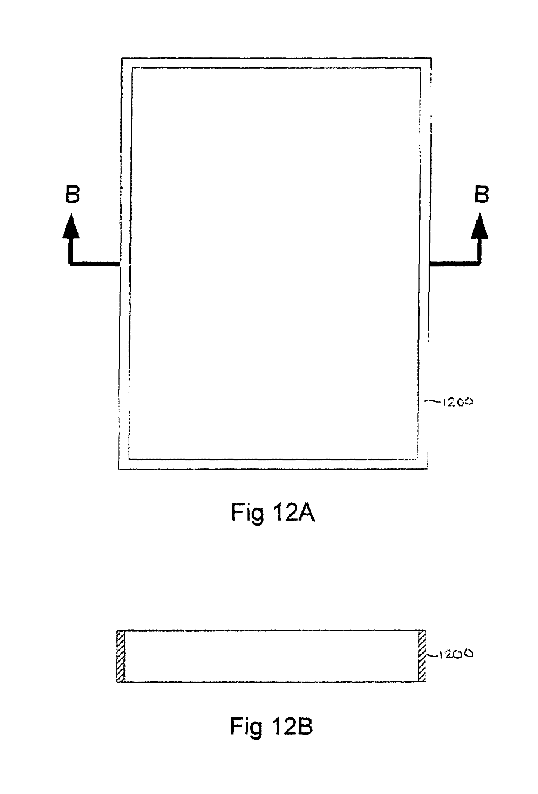

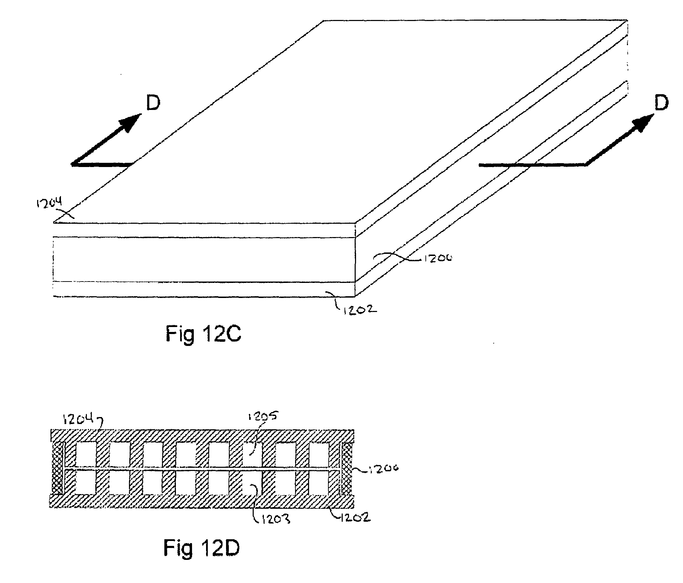

In another embodiment, the application provides a device for recovering cells comprising an alignment collar and separate collecting plate as illustrated in Figure 12. Accordingly, the application discloses a device for recovering cells from wells of a source plate comprising:
a) the source plate;
b) a collecting plate; and
c) a separate alignment collar;
wherein the alignment collar forms a bridge between the collecting plate and source plate; and
wherein the alignment collar fits outside the perimeter of the collecting plate and aligns the source plate, so that the source plate fits inside the alignment collar when the source plate, the collecting plate and the alignment collar of the device are assembled.

In one embodiment, the source plate that fits inside the alignment collar is a multi-well plate and the collecting plate is a multi-well plate, such that when the source plate is inverted, the cells from the wells of the source plate are collected into the corresponding wells of the collecting plate in the assembled device. In another embodiment, the assembled device is centrifuged. In yet another embodiment, the cells recovered from the assembled device are mammalian pluripotent stem cell aggregates such as mammalian embryonic stem cells aggregates or embryoid bodies. In another embodiment, the mammalian pluripotent stem cells are human.

The present application also relates to improved methods of reproducibly and efficiently generating cell aggregates from mammalian pluripotent stem cells such as mammalian embryonic stem cell aggregates or embryoid bodies from embryonic stem cells. The present application also relates to a method of employing controlled cell aggregate production to reproducibly and efficiently generate tissue-level organization within cell aggregates of mammalian pluripotent stem cells such as mammalian embryonic stem cell aggregates or embryoid bodies.

Applicant has determined several ways in which the efficiency of generating cell aggregates of mammalian pluripotent stem cells, such as mammalian embryonic stem cell aggregates or embryoid bodies from embryonic stem cells, may be improved. Accordingly, the application provides a method of generating cell aggregates from mammalian pluripotent stem cells comprising:
(1) preparing a population of undifferentiated mammalian pluripotent stem cells and differentiated cells by adding a factor to the mammalian pluripotent stem cells to promote cell differentiation in part of the population, or by separately culturing the mammalian pluripotent stem cells and differentiated cells; and
(2) preparing a mixture in suspension comprising the differentiated cells and undifferentiated cells of step (1); and
(3) forming cell aggregates from the mixture in step (2).

In another embodiment, the method of generating cell aggregates comprises:
(1) preparing a population of mammalian pluripotent stem cells with enhanced ability to form aggregates by adding a factor to the mammalian pluripotent stem cells to promote survival in single-cell suspension, prior to and/or while suspended as single cells; and
(2) preparing a suspension comprising the cells prepared in step (1); and
(3) forming cell aggregates from the mixture in step (2).

The method described above for generation of cell aggregates from mammalian pluripotent stem cells such as mammalian embryonic stem cells addresses the need in the art for consistent, efficient, scalable and reproducible formation of cell aggregates by employing various steps which the applicants have identified as significantly impacting the reproducibility of cell aggregate formation, resulting arbitrary numbers of regular, and uniform aggregates.

The application further describes a method that addresses the need in the art for efficient production of tissue-level order within cell aggregates from mammalian pluripotent stem cells, such as mammalian embryonic stem cell aggregates or embryoid bodies from mammalian embryonic stem cells. This method comprises the methods set out above and further comprises an additional step of maintaining the recovered cell aggregates from step (3) in suspension for an extended period; wherein the resulting cell aggregates exhibit tissue level organization within the cell aggregates. The method described substantially reduces the chaos and disorder characteristic of existent protocols and results in cell aggregates that exhibit tissue level organization within the cell aggregates, such as mammalian pluripotent stem cell aggregates, for example, embryonic stem cell aggregates or embryoid bodies. This higher order organization and aggregation is obtainable from single cell suspensions. In one embodiment, tissue level organization is visualized via confocal microscopy by assessing expression of marker proteins, such as E-cadherin and Oct4, and by assessing structural organization, such as columnar morphology and actin cytoskeleton.

The application further provides a microwell device comprising a fluid vessel having a bottom region. A microwell plate is at the bottom region. The microwell plate comprises an upper region defining an upper plane, and a plurality of microwells extending downwardly from the upper plane. Each of the microwells comprises an axis extending perpendicularly to the upper plane. Each of the microwells comprises a sidewall, and the sidewall of at least one of the microwells has at least one wall component extending inwardly towards the axis.

In some embodiments, the microwell plate is integral with the fluid vessel. For example, the fluid vessel may comprise a bottom surface, and the microwell plate may be stamped into the bottom surface. That is, the microwell plate may form a bottom surface of the fluid vessel. Alternately the microwell plate may be separately formed from the fluid vessel. That is, the fluid vessel may comprise a bottom surface, and the microwell plate may be seated on the bottom surface.

In some embodiments, the fluid vessel is selected from the group consisting of a vial, a beaker, a test-tube, a cryotube, a centrifuge tube, and a culture plate. In some embodiments, the fluid vessel is a well of a multi-well plate.

The application further provides a microwell device comprising a body comprising an upper region defining an upper plane. A plurality of microwells extend downwardly from the upper plane into the body. Each of the microwells comprises an axis extending perpendicularly to the upper plane. Each of the microwells comprises a sidewall. The sidewall of at least one of the microwells has at least one wall component extending inwardly towards the axis. A common fluid reservoir is provided above the upper plane.

In some embodiments, the microwell device further comprises a fluid vessel, and the body is provided at the bottom region of the fluid vessel. The fluid vessel can comprise at least one sidewall, and the sidewall can define the common fluid reservoir.

The application further provides a microwell device comprising a body comprising an upper region defining an upper plane. A plurality of microwells extend downwardly from the upper plane into the body. A particle retaining member is in communication with at least one of the microwells.

In some embodiments, the microwell device further comprises a common fluid reservoir provided above the upper plane.

In some embodiments the particle retaining member is a baffle. The baffle may be configured to modulate a flow of fluid in the at least one microwell.

In some embodiments, the particle retaining member is a filter member. The filter member may be configured to trap particles of greater than a selected size within the at least one of the microwells. The filter member may be a porous sheet extending across the at least one of the microwells, or a porous sheet extending across each of the microwells. The filter member may seat on an upper region of the body, and may be affixed or adhered to the upper region of the body.

In some embodiments, the filter member comprises a lid covering only a portion of the at least one microwell. The filter member may be affixed to the sidewall.

In some embodiments, the particle retaining member comprises a mesh. In some embodiments, the mesh may comprise pores of between 5 and 200 microns in diameter. In some particular embodiments, the mesh may comprise pores of between 37 and 75 microns in diameter. In some embodiments, the microwells are defined by a width at the upper plane, and the mesh comprises pores having a diameter of less than the width.

In some embodiments, the particle retaining member is positioned within the at least one of the microwells. In some embodiments, the particle retaining member is positioned below the upper plane. In some embodiments, the microwell device comprises a common fluid reservoir provided above the upper plane, and the particle retaining member is positioned in the common fluid reservoir.

In some embodiments, the microwells are defined by a width at the upper plane, and wherein the particle retaining member is spaced from the upper plane by a distance of less than the width. In some embodiments, the particle retaining member comprises a mesh having pores having a diameter, and the particle retaining member is spaced from the upper plane by a distance of less than the diameter. In some embodiments, the particle retaining member is spaced from the upper plane by a distance less than one cell diameter.

In some embodiments, each of the microwells comprises an axis extending perpendicularly to the upper plane, each of the microwells comprises a sidewall, and the sidewall of at least one of the microwells has at least one wall component extending inwardly towards the axis.

In some embodiments, the particle retaining member is configured to modulate a flow of fluid in the at least one of the microwells, and to trap particles of greater than a selected size within the at least one of the microwells.

In some embodiments, the particle retaining member comprises a baffle and a filter member separate from the baffle.

Other features and advantages of the present invention will become apparent from the following detailed description. It should be understood, however, that the detailed description and the specific examples while indicating preferred embodiments of the invention are given by way of illustration only, since various changes and modifications within the spirit and scope of the invention will become apparent to those skilled in the art from this detailed description.

### DRAWINGS

Figure 1A is a perspective illustration of a microwell plate in accordance with one embodiment of the present invention;

Figure 1B is a top plan view of the microwell plate of Figure 1A;

Figure 1C is a cross section taken along line C-C in Figure 1B;

Figure 1D is a perspective illustration showing a form and a negative usable to make a microwell plate of the present invention;

Figure 1E is a perspective illustration showing a method of making a form usable to make a microwell plate of the present invention; and

Figure 1F is a perspective illustration showing an alternate method of making a form usable to make a microwell plate of the present invention.

Figure 1G is a perspective illustration showing an alternate method of making a form usable to make a microwell plate of the present invention.

Figure 1H is a perspective illustration of a microwell device in accordance with an embodiment of the present invention;

Figure 1I is a cross section taken along line 1I-1I in Figure 1H;

Figure 1J is an enlarged view of the region shown in region 1J in Figure 1H;

Figure 1K is a top view of a microwell device in accordance with another embodiment of the present invention;

Figure 1L is a cross section taken along line 1 L-1 L in Figure 1K;

Figure 1M is a perspective illustration of a microwell device in accordance with an embodiment of the present invention;

Figure 1N is a cross section taken along line 1N-1N in Figure 1M;

Figure 1O is an enlarged view of the region shown in region 1O in Figure 1N;

Figure 1P is an enlarged view of the region shown in Box 1P in Figure 1O;

Figure 1Q is a top view of the region shown in Figure 1P;

Figure 1R is an alternate embodiment of the region shown in Figure 1P;

Figure 1S is a top view of the region shown in Figure 1R;

Figure 1T is an alternate embodiment of the cross section shown in Figure 1O;

Figure 1U is another alternate embodiment of the cross section shown in Figure 1O;

Figure 1V is another alternate embodiment of the cross section shown in Figure 1O;

Figure 1W is a top view of an alternate embodiment of a microwell device in accordance with the present invention;

Figure 1X is a partial cross section taken along line 1X-1X in Figure 1W.

Figure 2 shows the stages of construction of the device schematized in Figure 1. Each row depicts, from left to right, the microfabricated silicon wafer (with 400 micron scale bar), the PDMS negative after moulding on the silicon wafer, and the PDMS wells after replica moulding on the PDMS negative. Results for four sizes of wells are shown, with the 400, 200 and 100 micron wells extending to a point, while the 800 micron wells take the form of a truncated pyramid.

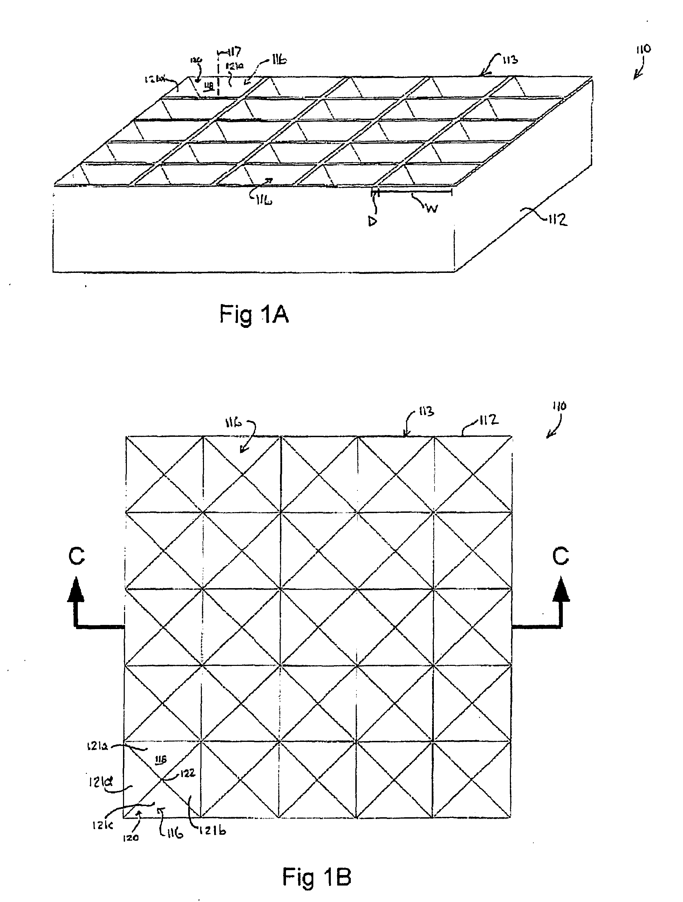

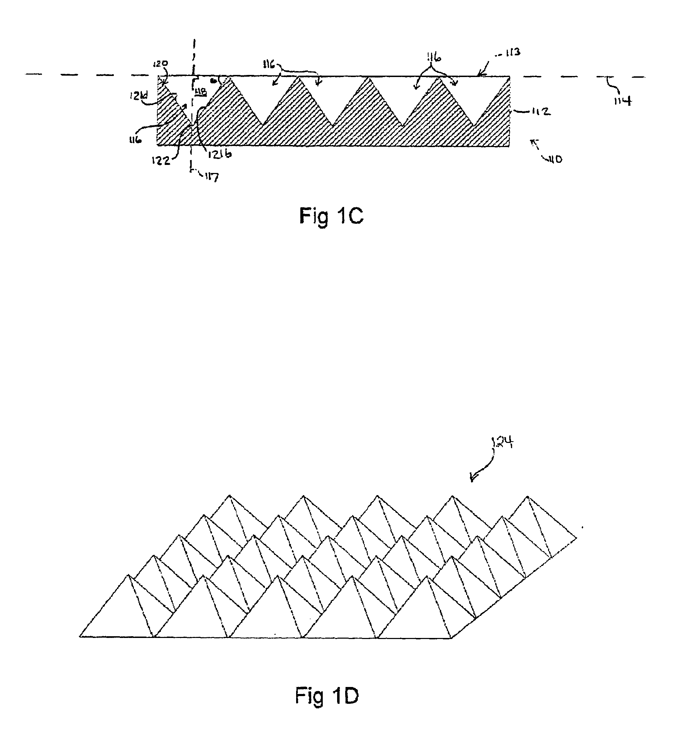

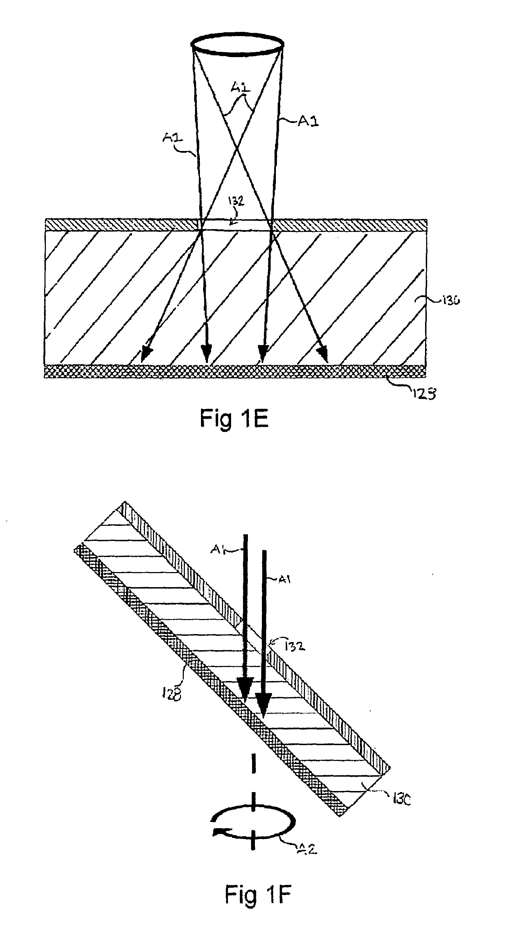

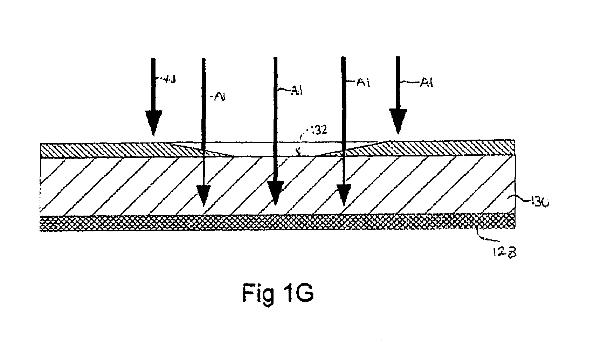

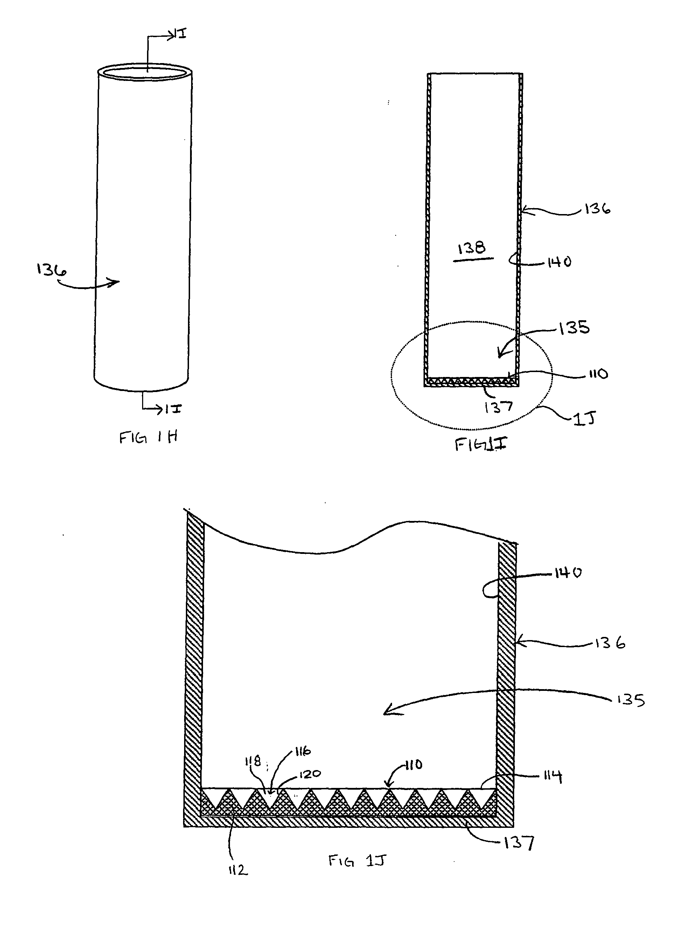

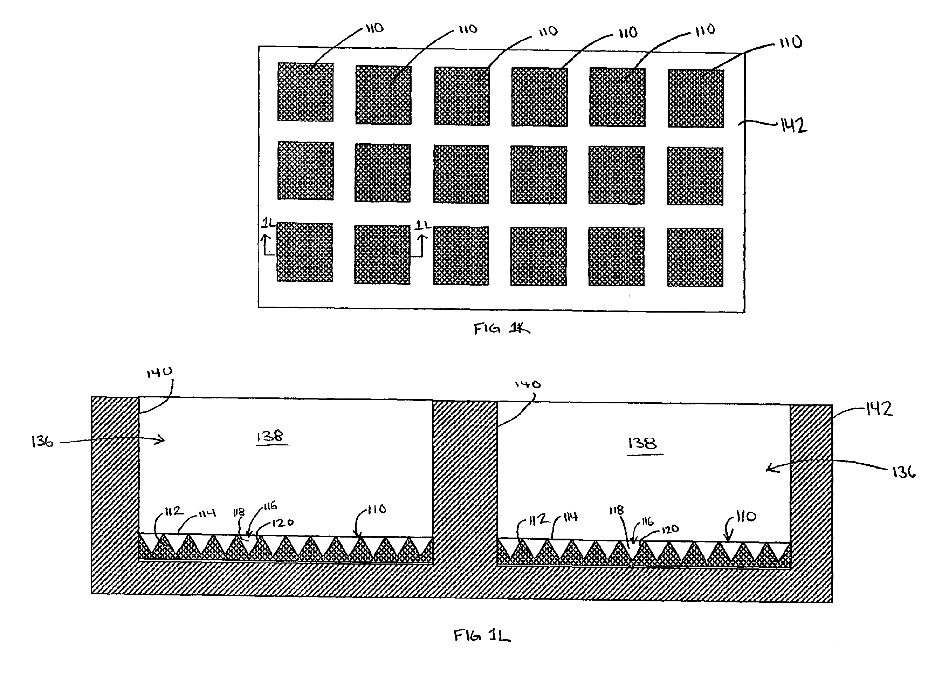

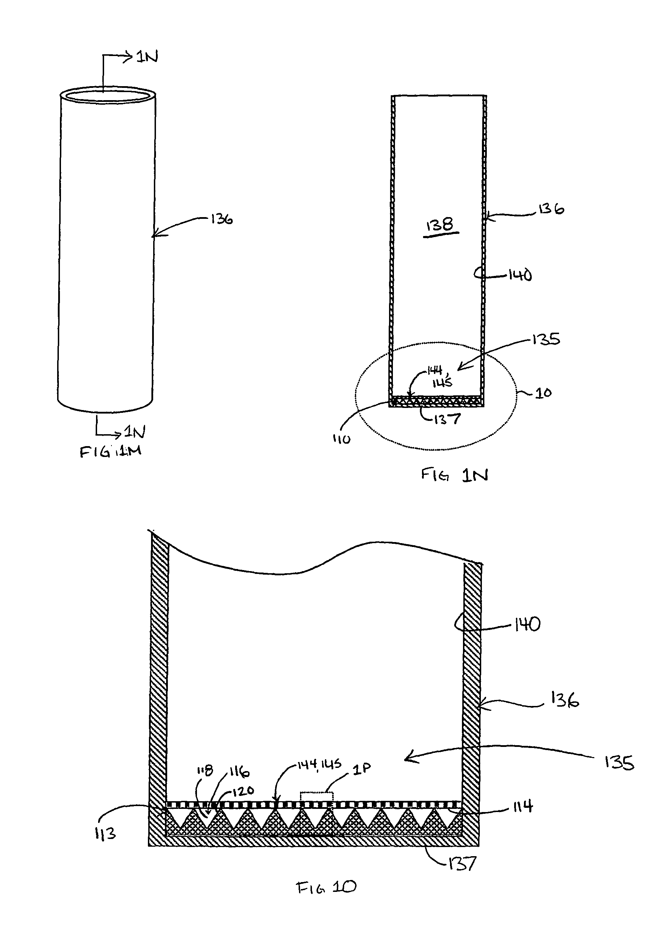

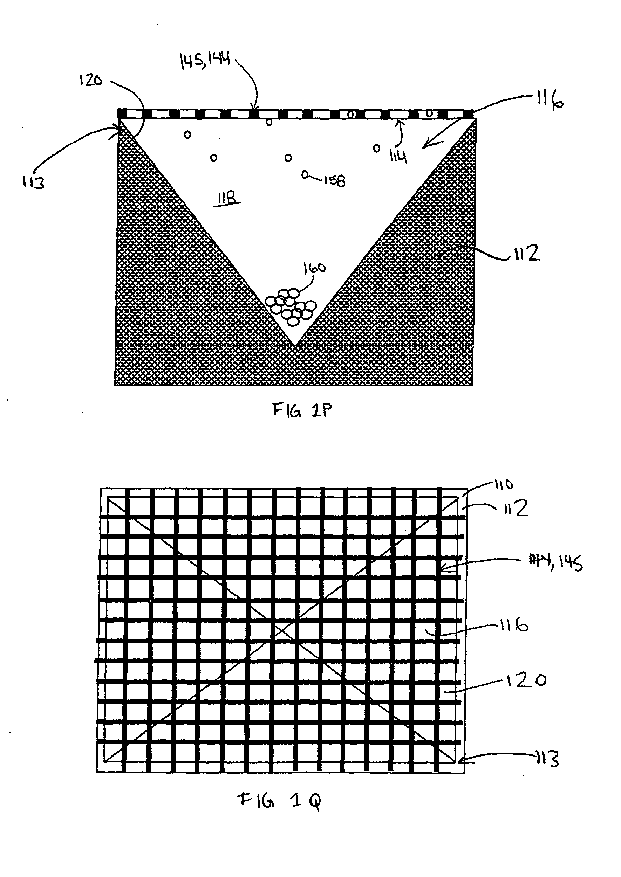

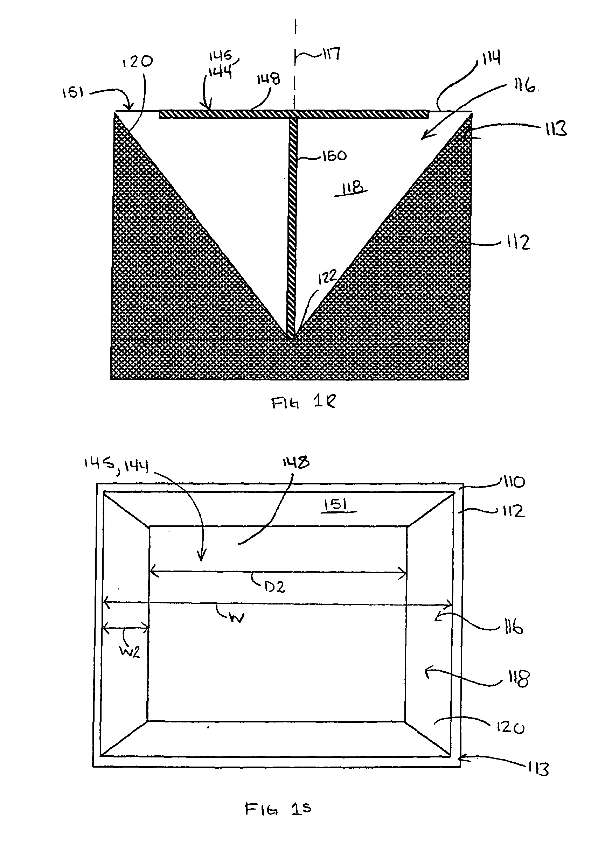

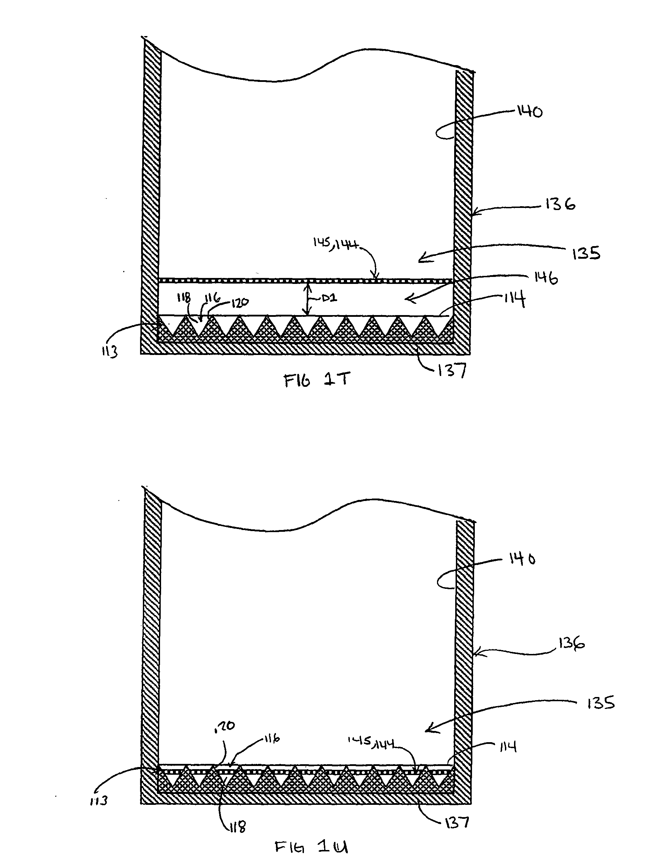

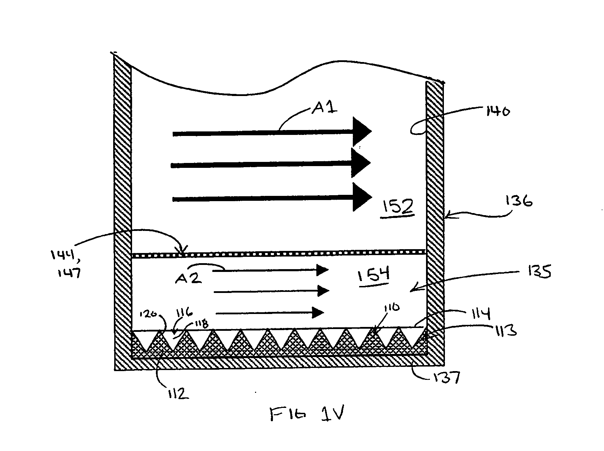

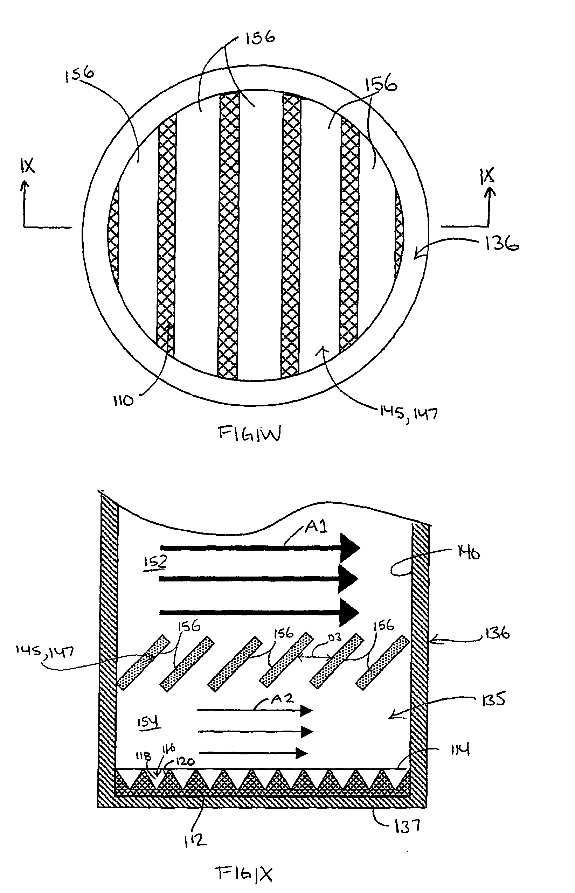

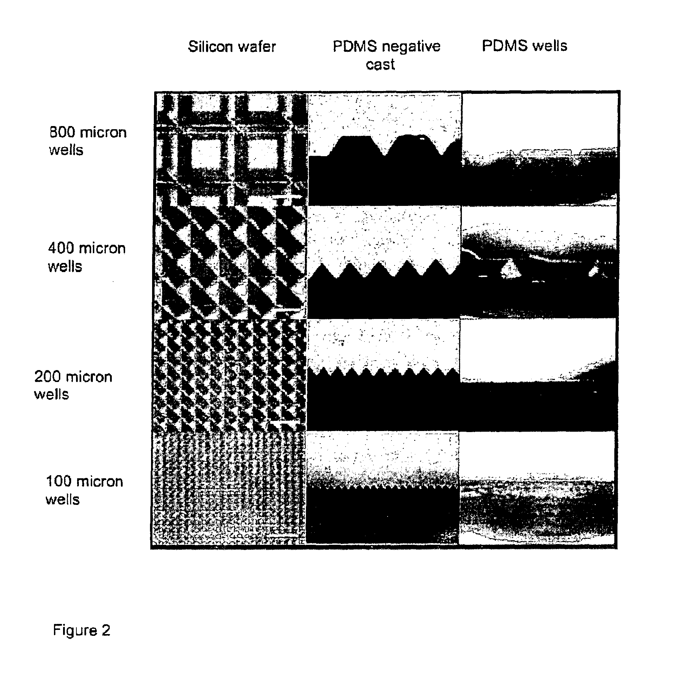

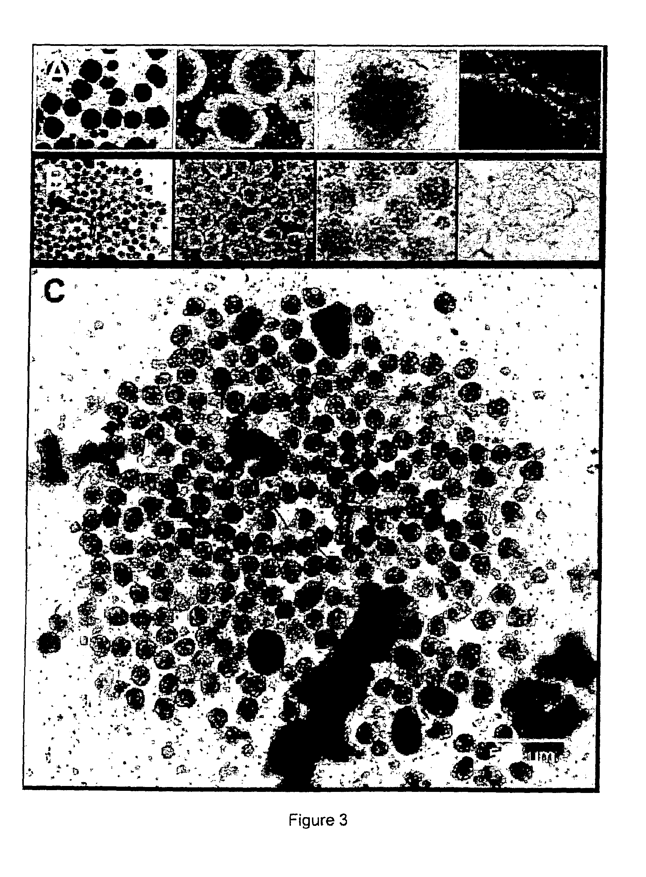

Figure 3 shows the generation of uniform hESC aggregates employing the device schematized in Figure 1 and depicted Figure 2. hESC cultured on Matrigel were treated with 10 µM Y-27632 and centrifuged into 800 micron (A) or 200 micron (B, C) PDMS microwells. Successive panels in (A) and (B) represent images taken at 4x, 10x, 20x, and 40x magnification. Panel C represents several images acquired at 4x magnification assembled together to show the number of aggregates retrieved from a single well containing an array of 200 micron microwells. Atypical aggregates are artefacts formed due to imperfections in the manually assembled prototype.

Figure 4 shows the generation of uniform hESC aggregates employing the device schematized in Figure 1 and depicted in Figure 2 without centrifugation. hESC cultured on Matrigel were treated with 10 µM Y-27632 and allowed to settle into 200 micron PDMS microwells and aggregated for 24 hours. Upper panel shows the aggregates in the microwells, lower panel shows aggregates after extraction.

Figure 5 shows the refeeding of aggregates in 400 µm wells. hESC cultured on MEF were pre-treated with serum containing medium for 48 hours, and centrifuged into 200 micron PDMS microwells. Upper panel shows the aggregates in the microwells after 24 hours. A portion of the aggregates were extracted, the remainder were refed in situ, lower panel shows aggregate development after an additional 48 hours in the wells.

Figure 6 shows the use of microwells as culture surface. hESC aggregates prepared in 96-well plate format were transferred into 800 µm PDMS microwells. Surface permits microscopic inspection while preventing aggregates from interacting directly. Scale bar is 200 µm.

Figure 7 shows the use of microwells to generate aggregates of non-ES cell types. MEF cells were loaded as a single-cell suspension into 400 µm wells at a ratio of 2,000 (A,D), 1,000 (B,E) or 500 (C,F) cells per microwell and centrifuged. Panels A through C show the aggregates in the microwells after 24 hours, panels D through F show the aggregates after subsequent extraction.

Figure 8 shows the use of microwells to generate aggregates of non-hES cell types. mESC containing GFP expressed under the control of the Brachyury promoter were aggregated in the device at 2,000 cells per microwell, and imaged immediately after extraction (upper panel), or after 6 days (lower panel) at which point GFP expression was active within a subregion of the aggregate.

Figure 9 shows the use of microwells for preparation of aggregates of tumor cells ("tumor spheroids"). HeLa tumor cells were aggregated in the device at 1,000 cells per microwell, incubated for 24 hours at 37 degrees / 5% CO2, extracted and imaged (Figure 28).

Figure 10 shows an image of negative micropyramids of 200 µm microwells in high temperature epoxy (top); an image of negative micropyramids of 400 µm microwells in epoxy; and 800 µm microwells hot-embossed directly into the plastic in the culture surface of a standard 6-well tissue culture plate.

Figure 11A is a top plan view of an embodiment of an integral alignment collar and collecting plate of the present invention;

Figure 11B is a cross section taken along line B-B in Figure 11A;

Figure 11C is a perspective illustration of the alignment collar and collecting plate of Figure 11A, showing an embodiment of a source plate positioned in the alignment collar;

Figure 11D is a cross section taken along line D-D in Figure 11C;

Figure 12A is a top plan view of an embodiment of a separate alignment collar and of the present invention;

Figure 12B is a cross section taken along line B-B in Figure 12A;

Figure 12C is a perspective illustration of the alignment collar of Figure 12A, showing an embodiment of a collecting plate and a source plate positioned in the alignment collar;

Figure 12D is a cross section taken along line D-D in Figure 12C;

Figure 13 shows hESC aggregates from hESC cultured on mouse embryonic fibroblasts (MEF), with (lower two rows) or without (upper two rows) 2 days pre-differentiation in differentiation medium [DM].

Figure 14 shows 4x magnification image of hESC aggregates from hESC cultured on MEF. Upper row: cells harvested at 90% confluence; Lower row:
cells harvested at 20% confluence. Left column: cells used as harvested; Right column: cells supplemented with additional MEF cells to a ratio of 1 MEF to every 2 human cells.

Figure 15 shows 10x magnification image of hESC aggregates from hESC cultured on MEF. Upper row: cells harvested at 90% confluence; Lower row: cells harvested at 20% confluence. Left column: cells used as harvested; Right column: cells supplemented with additional MEF cells to a ratio of 1 MEF to every 2 human cells.

Figure 16 shows cardiac differentiation from hESC aggregates, after 12 days in suspension culture in serum-containing medium. At bottom are frames from a video recording of a contractile aggregate, shown before (top panel) and during (bottom panel) a contraction. The middle panel is derived by subtracting the upper panel from the lower panel. The contraction trace (above) was generated by integrating the subtraction image derived from successive frames in the video over the area of contraction, and plotting a 5-point moving average.

Figure 17 shows aggregates generated from 10,000 cells each (top left panel) differentiated for 14 days (top center panel), where haematopoetic differentiation was detected by colony-forming assay (lower left panel), cytospin assay (lower center panel) and flow cytometric detection of the CD34 marker (right hand panels), from aggregates derived with and without BMP2 pre-differentiation.

Figure 18 shows hESC aggregates from hESC cultured on Matrigel, pre-differentiated with 25 ng / mL BMP2 (left pair of columns). By columns, from upper left, aggregates formed from 16,000 cells, then 8,000 then 4,000 and so on down to 125 cells at bottom right. Equivalent numbers of cells were processed in the same manner, in the absence of BMP2 pre-differentiation (right pair of columns).

Figure 19 shows hESC aggregates from hESC cultured on matrigel, with no pre-differentiation (upper panel), or pre-differentiated with 50 ng / mL BMP4 (lower panel).

Figure 20 shows hESC aggregates formed from 10,000 (top row), 2,000 (middle row) or 400 cells, recovered using the spin-out technique. These aggregates were subsequently followed for hematopoetic differentiation as shown in Figure 17

Figure 21 shows hESC aggregates transferred from one 96-well plate to another using an alignment collar.

Figure 22 shows aggregates formed from 2,000 hESC, imaged immediately after recovery (top panel), or after 1, 2, 3 or 4 days respectively (2nd panel to bottom panel). Note self organization into an ordered domain (white arrow) and a disordered domain (black arrow), and progressive encirclement of the ordered domain by the disordered domain over time (black line arrows).

Figure 23 shows a false-colour confocal image of day 2 hESC aggregate showing tissue level order. E-cadherin was preferentially expressed in the ordered domain, and laminin was preferentially expressed in the disordered domain.

Figure 24 shows a confocal image of day 5 hESC aggregate showing tissue level order. The ordered domain shows expression of the pluripotency marker Oct4, and here underlies the disordered domain, which expresses the endodermal marker GATA6 . Oct4 positive nuclei tended to align along the interface between the two tissue types, and a tendancy towards columnar morphology (white arrow), both characteristics of epiblast tissue.

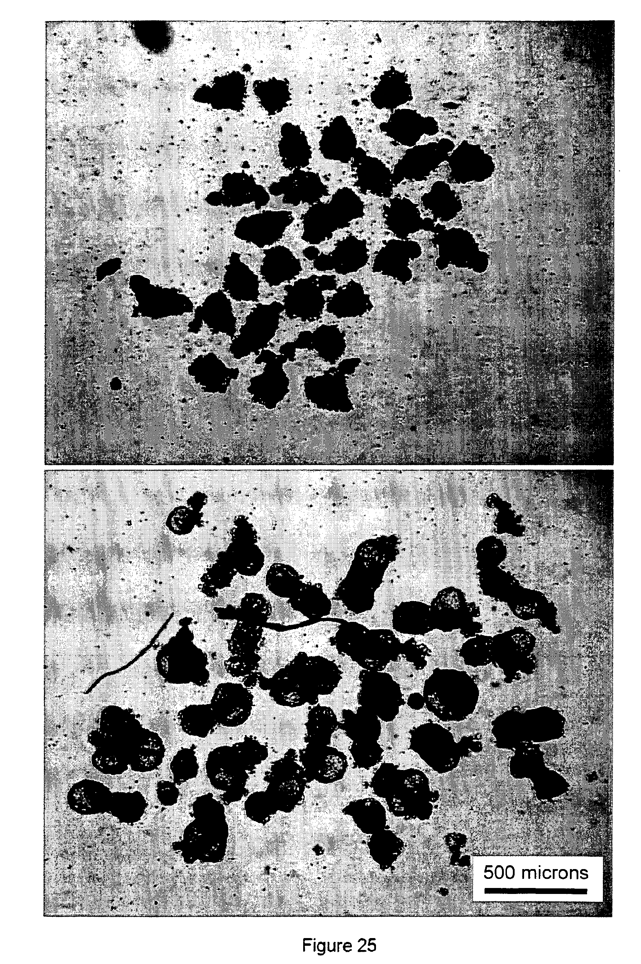

Figure 25: Aggregates may be formed by mixing multiple input populations: Images acquired 1 day after recovery of EB formed from 2,000 cells, either hESC as cultured (upper panel), or supplemented with cells differentiated via BMP2 in a 1:1 ratio (lower panel). Briefly the factor referred to is a growth factor such as bone morphogenetic protein (BMP) which is added to the mammalian pluripotent stem cells for a period of 24 to 120 hours. In another embodiment, the period is 48 hours. In another embodiment, BMP-2 and BMP-4 may be used as the BMP. In another embodiment, the concentration of BMP used may range from 3 ng/ml to 50 ng/ml. In a further embodiment, the concentration of BMP-2 used is 25 ng/ml. In yet a further embodiment, the concentration of BMP-4 used is 50 ng/ml BMP4.

Note the formation of cystic structures in the disordered domains of the aggregates in the lower panel.

Figure 26 shows the use of the ROCK inhibitor Y-27632 to enhance aggregate formation. hESC cultured on Matrigel without pre-differentiation (A) do not form aggregates. Pre-differentiation results in aggregate formation (B), however the size and symmetry of aggregates are improved by the addition of 10 µM Y-27632 in the absence (C) or presence (D) of pre-differentiation.

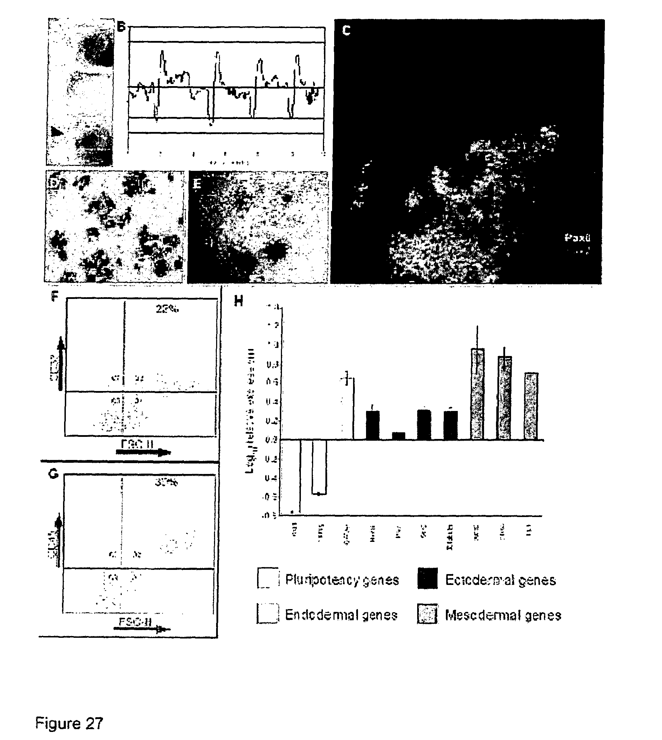

Figure 27 shows the differentiation to endoderm, ectoderm and mesoderm lineages including cardiac, hematopoetic and neural cells. Cardiac differentiation is from hEB differentiated for 12 days in suspension culture. in serum containing medium. A) two frames from a video recording of a contractile aggregate, shown before (Ai) and during (Aiii) a contraction. The middle panel is derived by subtracting the upper panel from the lower panel (Aii). The contraction trace (B) was generated by integrating the subtraction image derived from successive frames in the video over the area of contraction, and plotting a 5-point moving average. Neural rosettes (C) were observed, staining positive for Pax6, and Sox2. Hematopoetic differentiation was observed using Cytospin (D), CFC (E) and flow cytometric (F and G) assays. H. Quantitative RT-PCR results from aggregates differentiated for 4 days in suspension followed by an additional 3 days in adherent culture shows downregulation of pluripotency genes, and up-regulation of markers for endodermal, ectodermal and mesodermal lineages.

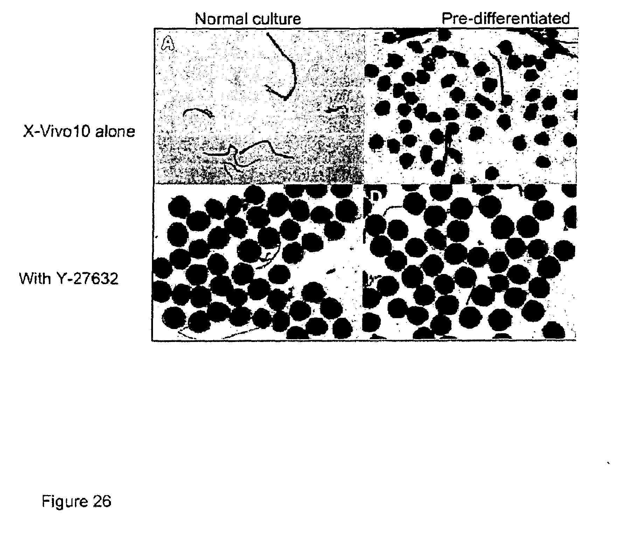

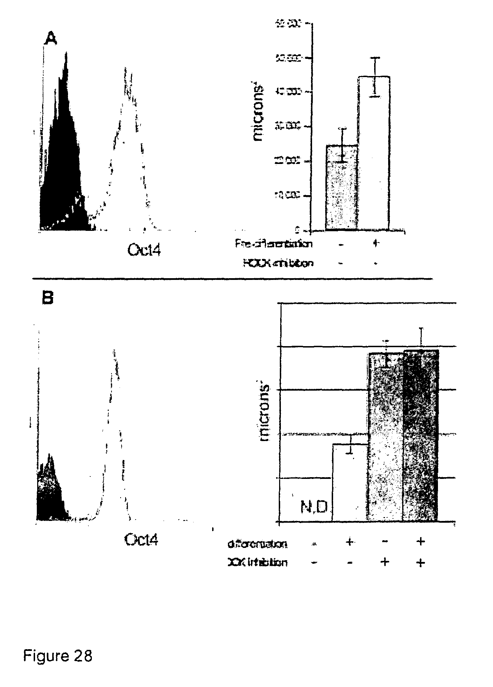

Figure 28 shows factors that control aggregate formation and stability: Pre-differentiation improves aggregate formation. (A) hESC cultured on mouse embryonic fibroblast (MEF) feeders were pre-differentiated with 20% serum for 72 hours prior to aggregate formation, resulting an overall reduction in population Oct4 expression (Figure 26A. right panel, dark line: standard maintenance culture; light line: pre-differentiated; black-fill: control (unstained) population. Aggregates formed from 2000 input cells were substantially larger with treatment (light bar) than without (dark bar). Y axis represents aggregate cross-sectional area in microns2, error bars represent one standard deviation. (B) shows that the ROCK inhibitor Y-27632 promotes aggregate stability. hESC cells cultured on Matrigel in MEF-conditioned medium with and without pre-differentiation were used to form spin in spin out (SISO) aggregates in the presence or absence of 10 µM Y-27632 (Figure 26B). In the absence of both, no aggregates were formed (N.D. - size not determined). With 48 hours predifferentiation in 20% serum, consistent aggregates were formed (light grey bar). When Y-27632 was added to the suspension of cells without (medium grey bar) or with (dark gray bar) pre-differentiation immediately prior to dispensing into the well plate, sizeable aggregates resulted.

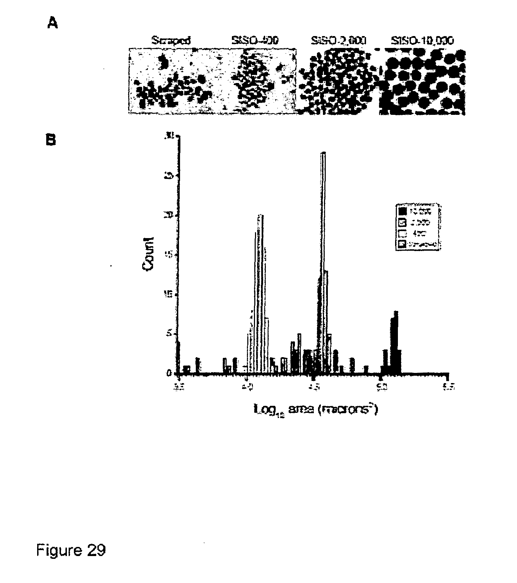

Figure 29 shows SISO-aggregation allows for the generation of size-controlled aggregates. (A) hEB were generated by scraping, and SISO-aggregates were generated from input populations of 400, 2,000 and 10,000 cells in 384-well plates, and recovered by centrifugation. After imaging in phase-contrast mode, images were thresholded and cross-sectional areas were calculated using the ImageJ software package. Values obtained were extremely consistent, with coefficients of variation of 0.09, 0.06 and 0.08 respectively, vs 0.72 for the scraped hEB. (B) The base-10 logarithm of cross sectional area is plotted on a histogram, demonstrating the clear separation between aggregate sizes and dramatic increase in size control over scraping techniques.

Figure 30 shows microwells juxtaposed with mesh: Panel (D) shows 400 and 800 micron microwells, juxtaposed to a mesh with 37-micron openings. Panel (E) shows the same device after introduction of cells (8,000, 2,000, 4,000 and 1,000 per microwell, respectively), immediately after introduction (upper row) and 24 hours later after the cells have formed aggregates (lower row).

Figure 31 shows 200 micron microwells with 37 micron mesh glued on with PDMS. Left and right panels show same fields of view, focused on cells (left) or mesh (right).

Figure 32 shows the #200 stainless steel mesh which has been heat welded to 800 micron polystyrene microwells (top) and the same microwells after removal of the steel mesh (bottom).

Figure 33 shows the 50 micron nylon mesh which has been heat welded to 800 micron polystyrene microwells (top) and the same microwells after removal of the nylon mesh (bottom).

Figure 34 shows a disc of microwell-containing silicone rubber inserted into a standard glass scintillation vial.

Figure 35 shows a disc of microwell-containing silicone rubber inserted into a modified 15 mL centrifuge tube.

Figure 36 shows a disc of microwell-containing silicone rubber inserted into a standard plastic cryovial.

Figure 37 shows solvent welding of 50 micron Nylon mesh onto 200 micron polystyrene microwells.

### DESCRIPTION OF VARIOUS EMBODIMENTS

Various apparatuses or processes will be described below to provide an example of an embodiment of each claimed invention. No embodiment described below limits any claimed invention and any claimed invention may cover processes or apparatuses that are not described below. The claimed inventions are not limited to apparatuses or processes having all of the features of any one apparatus or process described below or to features common to multiple or all of the apparatuses described below. It is possible that an apparatus or process described below is not an embodiment of any claimed invention. The applicants, inventors or owners reserve all rights that they may have in any invention disclosed in an apparatus or process described below that is not claimed in this document, for example the right to claim such an invention in a continuing application and do not intend to abandon, disclaim or dedicate to the public any such invention by its disclosure in this document.

The present application relates to devices and methods that are useful in preparing cell aggregates.

In one embodiment, the present invention provides a microwell device comprising:

a) a body comprising an upper region defining an upper plane;

b) a plurality of wells extending downwardly from the upper plane into the body;

c) each of the wells comprising an axis extending perpendicularly to the upper plane; and

d) each of the wells comprising a sidewall, the sidewall of at least one of the wells having at least one wall component extending inwardly towards the axis.

In some further embodiments, the microwell device comprises minimal spacing between the microwells such that substantially all of the cells falling on the surface will land in a microwell and participate in aggregate formation

Referring to Figures 1A to 1F, an embodiment of a device in accordance with one aspect of the invention is shown. The device is a microwell plate 110 usable for the formation of cell aggregates according to the techniques described herein.

Referring to Figures 1A to 1C, plate 110 comprises a body 112, which in the embodiment shown is substantially rectangular cubic. In alternate embodiments, the body 112 may be, for example, square cubic, or another shape. Body 112 comprises an upper region 113, which defines an upper plane 114 (shown in Figure 1C).

A plurality of wells 116 extend downwardly from upper plane 114 into body 112. Each of the wells comprises an axis 117 extending therethrough, and each axis 117 is perpendicular to the upper plane 114. In the embodiments shown, axis 117 is an axis of symmetry; however, in alternate embodiments, axis 117 may not be an axis of symmetry.

Referring still to Figures 1A to 1C, each well 116 comprises a volume 118, which is defined by a sidewall 120. In the embodiment shown, each sidewall 120 comprises four wall components 121a, 121b, 121c, and 121d. In an alternate embodiment, each sidewall 120 may comprise, for example, a single rounded wall component 121. In yet further embodiments, each sidewall 120 may comprise an alternate number of wall components 121, and the invention is not limited in this regard.

At least one of the wells is configured such that at least one wall component 121 thereof extends inwardly towards axis 117. That is, at least one of the wall components 121 of at least one of the wells is at an angle θ of less than 90° with respect to the upper plane 114. For example, in the embodiment shown, each well comprises four wall components 121a, 121b, 121c, and 121d, which are each at an angle θ of less than 90° with respect to upper plane 114. In an alternate embodiment, only some or only one of the wall components 121a, 121b, 121c, and 21d, may be at an angle of less than 90° with respect to upper plane 114. For example, wall components 121a and 121c, may be an angle θ of less than 90° with respect to upper plane 114, and wall components 121b and 121d may be at an angle of 90° with respect to upper plane 114. In yet another alternate embodiment, wherein wells 116 comprise a single rounded wall component, the single rounded wall component may be at an angle of less than 90o with respect to upper plane 114.

The angle θ between the wall component(s) 121 and the upper plane 114 may vary depending on the particular embodiment. In the embodiment shown, the angle θ is about 54.7°. In other embodiments the angle θ may be between about 20° and about 80°. Providing wells 116 with wall components 121 that are at an angle with respect to upper plane 114 may cause the aspect ratio of the aggregates produced to be independent of cell number over a wide range of aggregate sizes that fit within a well.

In the embodiments shown, each wall component 121 is substantially straight, and the slope of each wall component 121 is substantially constant along the height thereof. In alternate embodiments (not shown), one or more of the wall components 121 may be curved, and thus slope of the wall component 121 may vary along the height thereof. Furthermore, in the embodiments shown, the wall components 121 meet at a lower apex 122. Accordingly, for a well 116 of given dimensions, any number of cells, from 2 up to the volumetric capacity of the well 116, will be forced to contact one another when deposited into the well 116. In alternate embodiments however, each well 116 may further comprise a base wall (not shown), extending between or within the one or more wall components.

Volume 118 may be of a variety of shapes, depending on the number of wall components 121 of each sidewall, the angle of each wall component 121 with respect to upper plane 114, and the shape of each wall component 121. For example, in the embodiment shown, each wall component 121 is substantially triangular. Accordingly, each volume 118 is substantially square pyramidal. In an alternate embodiment (not shown), wherein each well 116 comprises a single rounded wall component 121, the volume may be substantially conical. In yet alternate embodiments, wherein wells 116 comprise a base wall, volume 118 may be, for example, substantially frusto-pyramidal, or frusto-conical.

Wells 116 may be of a variety of sizes, depending on the particular embodiment, and the intended use of plate 110. For example wells 116 may have a dimension of about 100 microns, 200 microns, 400 microns, or about 800 microns. In the embodiment shown, the term 'dimension' refers to the width W of the wells 116 at upper plane 114. However, in alternate embodiments, wherein wells 116 are of a different shape, 'dimension' may refer to a diameter, or to a length at upper plane 114, for example. Embodiments having wells of 100 microns may be used to make aggregates of, for example, about 10 cells. Embodiments having wells of 200 microns may be used to make aggregates of, for example, about 100 cells. Embodiments having wells of 200 microns may be used to make aggregates of, for example, about 2000 cells.

In some embodiments an anti-adherent coating (such as pluronic acid) may be applied to sidewalls 120. However, in some embodiments, due to the angle of wall component(s) 121, such a coating may not be necessary to promote aggregation. In yet other embodiments, sidewalls 120 may be Matrigel coated.

The number of wells provided on given plate may vary depending on the particular embodiment. In the embodiment shown, plate 110 comprises 25 wells. In alternate embodiments, plate 110 may comprise, for example, 6,000 wells, or another desired number of wells

In the embodiment shown, the wells 116 are arranged in an array. In some embodiments, the array is closely packed. That is, each well 116 is defined by a width W at upper plane 114, and the distance D between each well 116 is less than width W. In some embodiments, the distance D between the wells may be less than five cell diameters. In some particular embodiments, the distance D between each well 116 may be less than 100 microns. In further embodiments, the distance D between each well 116 may be less than 10 microns. Such embodiments, wherein the array is closely packed, may aid in forcing the cells to be spun into the wells 116.

Although in the embodiment shown, the wells 116 of plate 110 are identically configured (i.e. plate 110 comprises 25 identical wells), it will be appreciated that plate 110 may comprise a plurality of wells that are not identical. For example, plate 10 may comprise a plurality of pyramidal wells, and a plurality of conical wells. Furthermore, in some embodiments, only one or only some of the wells may be provided with a wall component that extends inwardly towards axis 117.

Plate 110 may be manufactured by a variety of methods. In one embodiment, as shown in Figure 1D, plate 110 may be manufactured by making a form or master, which is then used to create a negative 124, which used to form the plate 110.

In one embodiment, the form or master may be manufactured by etching of silicone. For example, a crystalline silicone wafer may be etched with potassium hydroxide to form wells 116. In such an embodiment, if the silicone wafer has a crystal orientation of 1-0-0, and is masked with silicone nitride having a regularly spaced array of openings, the etching may form square pyramidal well having an angle of 54.7° with respect to upper plane 114. If the etching is allowed to proceed to completion, the wall components 121 of each well will meet at lower apex 122, as described hereinabove. This process is called anisotropic wet etching using potassium hydroxide. If the etching is terminated prior to completion, the wells may be substantially frusto-pyramidal, having a base wall. From the silicone master, a PDMS (polydimethylsiloxae) negative may be made, which may then be used to mold a PDMS plate.

In an alternate embodiment, the form or master may be made by photolithography. For example, referring to Figure 1E, a silicone wafer 128 coated with a photoresist material 130 may be masked with a regularly spaced array of openings 132. The photoresist material may then be exposed to diffused incident light, indicated by arrows A1, such that the light passes through the mask at an angle. If the openings 132 are circular, due to the angle of the incident light, the volume of the photoresist stabilized by the light will be substantially conical. Areas of reduced light exposure may also occur at the periphery of the exposed region, resulting in reduced stabilization of the photoresist, and will also contribute to the generation of non-vertical-sidewalls.. Alternatively, referring to Figure 1F, the silicone wafer and photoresist material may be positioned at an angle with respect to incoming parallel incident light, and may be rotated in a direction indicated by arrow A2. Again, if the openings 132 are circular, the volume of photoresist stabilized by the light will be substantially conical. In yet another embodiment, as shown in Figure 1G, a shaded mask 134 may be used (e.g. of gradually increasing thickness, or of gradually increasing darkness), such that the region of photoresist 130 directly beneath opening 132 is exposed to the highest level of light, and the region of photoresist around opening 132 is exposed to a lower level of light. Again, if the openings 132 are circular, and the mask is gradually shaded to let less light through as the distance from openings 132 increases, the volume of photoresist stabilized by the light will be substantially conical. In any of these embodiments, after the photoresist has been selectively stabilized, the un-stabilized portion may be washed off, and the remaining photoresist may be used to form a PDMS negative, which is used to mold a PDMS plate.

In yet another alternate embodiment, plate 110 may be manufactured by individually drilling wells 116 out of a silicone wafer, or another substrate.

Referring to Figures 1H-L, in some embodiments, plate 110 is provided at the bottom region 135 of a fluid vessel 136 This fluid vessel permits a desired volume of liquid to be retained directly above plate 110. The vessel may also have outside dimensions compatible with other pieces of equipment, for example centrifuge rotors, automated fluid handling devices and so on, so as to permit the use of plate 110 in conjunction with said equipment. The vessel may also have a lid, screw-cap or other closure to isolate or partially isolate (for example as in a closure with a gas-permeable filter, or a closure that may be incompletely closed) the contents of the vessel from their environment. The vessel may protect its contents from the environment, and it may protect the environment from the contents (for example in the case of biohazardous materials being used). The vessel may be of material chosen to have desired properties such as chemical resistance or permeability. The vessel may be tall enough to contain a fluid column in which differential sedimentation rates allow separation of centrifuged objects such as cells into distinguishable populations during centrifugation, resulting in inhomogeneous aggregates. The vessel may be short enough to prevent separation of centrifuged objects. In such embodiments the fluid vessel 136 may provide a common fluid reservoir 138 above the microwells 116 of the plate 110. For example, the fluid vessel 136 may comprise a sidewall 140, and the sidewall 140 may define the common fluid reservoir 138. For example, as shown in Figures 1H-J, plate 110 is seated on the bottom surface 137 of a vial 136, and the sidewalls 140 of the vial 136 define a common fluid reservoir 138 above the microwells 116. In other embodiments, plate 110 may be provided at the bottom region of a test tube, a culture plate, a scintillation vial or other vial, a beaker, a cryotube, a centrifuge tube, or another type of fluid vessel. In one particular embodiment, as shown in Figures 1K and 1L, a plurality of plates 110 are provided, and each is positioned at the bottom region of a well 136 of a multi-well plate 142.

In the embodiments shown, the plate 110 is separately formed from the fluid vessel 136. For example, the plate 110 may be formed as described hereinabove with respect to Figures 1D to 1G, and may be inserted into the fluid vessel 136 and positioned at the bottom region 135 of the fluid vessel such that it is seated on the bottom surface 137 of the fluid vessel 136. Optionally, the plate 110 may be adhered or affixed to the bottom surface 137

In other embodiments, the plate 110 may be integral with the fluid vessel 136. For example, the fluid vessel 136 may comprise a multi-well plate 142 that is fabricated from a plastic, and the microwells 116 may be stamped into the bottom surface 137 of the each of the wells of the multi well plate.

Referring now to Figures 1M to 1X, in some embodiments, a particle retaining member 144 is provided in fluid communication with one or more of the microwells 116. The particle retaining member 144 retains or aids in retaining particles, for example cellular aggregates, in the microwells 116 or adjacent the microwells 116. For example, as will be described in further detail hereinbelow, the particle retaining member 144 may be a filter member which is configured to prevent particles of larger than a selected size from passing therethrough or thereby, and therefore may be used to physically trap the particles within or adjacent the microwells 116. Alternately, the particle retaining member 144 may be a baffle configured to modulate a flow of fluid in or adjacent the microwells and thereby prevent, minimize, or reduce sloshing of fluid and particles out of the microwells.

In the embodiments shown in Figures 1M-1Q, the particle retaining member 144 is in communication with each of the microwells 116. In the embodiment shown in Figures 1R and 1S, the particle retaining member 144 is in communication with only one of the microwells 116. In alternate examples, the particle retaining member 144 may be in communication with only one or only some of the microwells 144, or a plurality of particle retaining members 144 may be provided, each of which is in communication with one or more of the microwells 144.

As used herein, the term 'in communication' indicates that the material passing through (e.g. in the case of a mesh, as will be described hereinbelow) or by (e.g. in the case of a lid, as will be described hereinbelow) the particle retaining member 144 may reach the microwell(s) 116, either directly, for example by passing through the particle retaining member 144 directly into the microwell(s) 116, or indirectly, for example by passing through the particle retaining member 144 into a common fluid reservoir 138, and from the common fluid reservoir 138 into the microwell(s) 116.

Referring now to Figures 1M to 1U, as mentioned hereinabove, in some embodiments, the particle retaining member 144 is a filter member 145 configured to trap particles of greater than a selected size within or adjacent the microwell(s) 116, and additionally, to allow particles of less than a selected size to enter and exit the microwell(s) 116. For example, the filter member 145 may be configured to allow single cells or small cell clumps (shown schematically by reference numerals 158 in Figure 1 P) to pass therethrough or thereby and enter and exit the microwell(s) 116, but to prevent larger cell clumps or cell aggregates (shown schematically by reference numerals 160 in Figure 1 P) from passing therethrough. Accordingly, the filter member 145 may serve to trap cell aggregates in or adjacent the microwells.

In some embodiments, in order to trap particles of greater than a selected size within or adjacent the microwell(s) 116, the filter member 145 may comprise a porous sheet, as is shown in Figures 1M - 1Q. For example, the filter member 145 may comprise a woven or non-woven mesh. The mesh may be, for example, stainless steel, polystyrene, nylon, degradable sutures, or specialized "smart" and engineered materials, or another material that is compatible with cells in the microwell(s) 116. Such "smart" materials may be configured to release particles after a certain amount of time, or to degrade at a selected rate. In some particular examples, the mesh may have a pore size of between 37 and 75 microns. For example, the mesh may have a pore size of about 50 microns. Alternately, the mesh may have a pore size of about 37 microns. A pore size of of between 37 and 75 microns may generally allow single cells and small cell clumps to pass therethrough, but will prevent larger cell clumps or cell aggregates from passing therethrough. In other examples, the pore size may be as low as 5 microns, or up to 200 microns or higher.

In such embodiments, wherein the filter member 145 is a porous sheet, the filter member may seat on the upper region 113 of the plate 110, and/or may be adhered or affixed to the plate 110. For example, the filter member 145 may be adhered to the upper region 113 of the plate using an adhesive, or by melting a portion of the upper region (for example by using heat or a solvent), applying the filter member 145 to the upper region 113, and then hardening the portion of the upper region 113. Additionally or alternately, the filter member 145 may be affixed to the plate 110 by seating a weight on the filter member to secure it to the plate, or by using a clamping member (not shown). In such embodiments, wherein the filter member 145 is at the upper plane of the plate, the filter member may trap the particles within the microwells.

In some embodiments, wherein in the filter member 145 is adhered or affixed to the plate 110, it may also be configured to be removable from the plate 110. For example, it may be manually removable from the plate 110, by applying force to remove it. Alterntely, a solvent may be used to remove the filter member 145 from the plate 110. Alternately, the filter member 145 may be degradable, and may slowly detach from the plate 110 over time.

In alternate embodiments, the porous sheet may be positioned above the upper plane 114. For example, as shown in Figure 1T, the porous sheet is positioned in the common fluid reservoir 138, and is spaced from the upper plane 114 by a distance D1. The porous sheet may be, for example, affixed to the fluid reservoir 138, or supported by a support (not shown). In such embodiments, wherein the filter member 145 is above the upper plane 114 of the plate 110, the filter member 145 may trap the particles in a region 146 adjacent the microwells 116, rather than within the microwells 116. Alternately, the porous sheet may be positioned below the upper plane 114. For example, as shown in Figure 1U, the porous sheet is positioned within the microwells 116, and is spaced from the upper plane 114 by a distance D1 (not labeled). The value of the distance D1 (in a direction either below the upper plane or above the upper plane) may vary depending on the particular embodiment. In some embodiments, D1 may be less than the dimension of the microwell 116. In some embodiments, D1 may be less than the pore size of the mesh. In some embodiments, D1 may be less than one cell diameter.

In some embodiments (not shown), the filter member may comprise more than one porous sheet. For example, the filter member may comprise a first mesh of a first pore size, and a second mesh of a different pore size.

In other embodiments, in order to trap particles of greater than a selected size within or adjacent the microwells 116, the filter member 144 may comprise a lid that covers only a portion of the microwell 116. For example, referring to Figures 1R and 1S, a lid 148 is shown, which has a dimension D2 less than the dimension W of the microwell 116. Accordingly, an annular gap 151 is formed between the microwell 116 and the lid 148, through which material may pass. The annular gap 151 may, for example, have a width W2 of between 5 and 200 microns, and more particularly, about 37 microns. Accordingly, single cells and small cell clumps may pass therethrough, but larger cell clumps or cell aggregates may be prevented from passing therethrough. In the embodiment shown, the lid 148 is mounted on a support 150, which is affixed to a sidewall 120 of the microwell 116.

In the embodiment shown, the lid 148 is positioned at the upper plane 114, and accordingly traps the particles within the microwells 116. However, in alternate embodiments, the lid 148 may be above the upper plane 114, and therefore may trap the particles in a region adjacent the microwells 116, or may be below the upper plane, and may be spaced from the upper plane by a distance D1, as described hereinabove.

In some embodiments, as mentioned hereinabove, in addition to or as an alternative to being a filter member 145 configured order to trap particles of greater than a selected size within or adjacent the microwells 116, the particle retaining member may be a baffle 147 configured to modulate a flow of fluid in the at least one of the microwells 116. That is, the particle retaining member 144 may minimize or reduce the transmission of turbulent flow from a region above the particle retaining member 144 to the region below the particle retaining member 144, or to protect the region below the particle retaining member 144 from high flow rates that occur in the region above the particle retaining member 144. Accordingly, the particle retaining member 144 may prevent or minimize sloshing of particles such as cellular aggregates out of the microwells 116.

For example, referring to Figure 1V, in use, plate 116 may be provided at the bottom of a fluid vessel 136, which is filled with a fluid. It may be desired to change or add to the fluid, to replenish the fluid, or to move the vessel 136, all of which may create turbulence or high flow rates in the vessel 136. In order to minimize or reduce the amount of disturbance this causes to the contents of the microwells 116, a particle retaining member 144 in the form of a baffle 147 may be provided in the vessel 136. The particle retaining member 144 may protect the region 154 below the filter member from these high flow rates (indicated by arrows A1), such that lower flow rates (indicated by arrows A2) occur in region 154 than in the region 152 above the particle retaining member 144.

In some embodiments, a filter member 145 as described with respect to Figures 1M to 1U may also serve as a baffle 147. That is, filter member 145 may both trap particles of greater than a selected size within or adjacent the the microwells 116, and modulate a flow of fluid in the at least one of the microwells 116. In other embodiments, the particle retaining member 144 may only be a baffle 147, without trapping particles of greater than a selected size within the or adjacent microwells 116. For example, referring to Figures 1W and 1X, the particle retaining member 144 comprises a plurality of angled slats 156 extending across the fluid reservoir 138, which form a baffle 147. The slats 156 are spaced apart by a distance D3, which is larger than the dimension W of the microwells. Accordingly, the slats 156 would not trap particles within the microwells 116. However, the slats would protect the contents of the microwells 116 from the high flow rates occurring above the slats 156.

In some embodiments (not shown), the particle retaining member 144 can include both a filter member 145, and a baffle 147. For example, a filter member 147 may be seated on the upper plane 114, and a baffle 147 may be positioned above the upper plane 114.

It will be appreciated that although the particle retaining member 144 serves to trap the particles within or adjacent the microwells 116 and/or to modulate a flow of fluid in or adjacent the microwells 116, some of the particles may still exit the microwells 116 or the region adjacent the microwells 116. That is, the particle retaining member may retain only some of the particles in or adjacent the microwells.

It will be appreciated that although the particle retaining member 144 has been described with reference to a microwell plate 110 having microwells 116 wherein at least one wall component 121 extends inwardly towards an axis 117, the particle retaining member 114 may be used with microwell plates having microwells wherein each of the wall components 121 are parallel to axis 117, or wherein the wall components 121 have any other configuration.

Further, it will be appreciated that although the particle retaining member 144 is shown in use when the microwell plate 110 is within a fluid vessel, in some embodiments, the, particle retaining member may be useful when the microwell plate 110 is not within a fluid vessel. For example, the particle retaining member 144 may prevent sloshing of fluid out of the microwells 116 when the microwell plate 110 is being transported.

In some embodiments, particularly when the fluid retaining member 144 is within a microwell 116 or at the upper plane 114, the fluid retaining member 114 may additionally serve as a culture surface for cells in the microwell 116.

As mentioned previously, the device or plate shown in Figure 1 is useful in preparing cell aggregates. Accordingly, the present application provides a use of the microwell device to prepare cell aggregates. The method also provides method of preparing cell aggregates comprising:

1) incubating cells on a device comprising:

a) a body comprising an upper region defining an upper plane;

b) a plurality of wells extending downwardly from the upper plane into the body

c) each of the wells comprising an axis extending perpendicularly to the upper plane; and

d) each of the wells comprising a sidewall, the sidewall of at least one of the wells having at least one wall component extending inwardly towards the axis,

wherein cell aggregates form in the wells of the device; and optionally

2) recovering the cell aggregates.

The shape of the wells of the above device, as well as the high density of wells in the device, force the cells to aggregate in the wells.

In one embodiment, the cells are centrifuged into the wells of the device. In another embodiment, the cells settle into the wells of the device by the force of gravity.

The cells can be any type of cell that can form cell aggregates including, without limitation, stem cells (including adult stem cells, embryonic stem cells, and "iPS" or induced pluripotent stem cells), fibroblasts, cardiomyocytes, endothelial cells, pancreatic islet cells, chondrocytes, .stroma) cells, hepatocytes, neural cells, cells of early germ layers (e.g. endoderm, ectoderm, mesoderm), and tumor cells, as well as combinations of two or more cell types. In a preferred embodiment, the cells are stem cells such as mammalian pluripotent stem cells including human embryonic stem cells (hESC).

In one embodiment, the cells are stem cells and are incubated with a cell survival factor that enhances the capacity of the cells to form aggregates. The cell survival factor can be any factor that enhances cell survival or reduces cell death. In one embodiment, the cell survival factor is an inhibitor of apoptosis such as a protein that inhibits a caspase such as caspase 3, 7 and/or 9. Inhibitors of apoptosis are well known in the art and include vad FMK. In a specific embodiment, the cell survival factor is the ROCK inhibitor Y-27632.

The cell aggregates may comprise from 2 to 100,000 cells. The larger the aggregates the larger the microwells used. In one embodiment, aggregates of at least 10 cells are made and the microwells are at least 200 microns. In another embodiment, aggregates of at least 1,000 cells, preferably at least 2,000 cells, are made and the microwells are at least 400 microns. In yet another embodiment, aggregates of at least 10,000 cell or at least 100,000 cells are made in 800 micron wells.

The aggregates may be maintained in the microwell device or they may be recovered from the device.

This application further describes a method of recovering cell aggregates. In one embodiment, the cell aggregates are recovered by pipetting. In another embodiment, the cell aggregates are recovered from wells of a source plate comprising a method involving centrifugation of an assembled device, wherein the source plate is inverted; and wherein the assembled device comprises:

a) the source plate; and

b) a single unit further comprising an alignment collar attached to a collecting plate;

wherein the alignment collar is attached perpendicularly to a base of the collecting plate; and

wherein the alignment collar of the single unit fits outside the perimeter of the source plate and aligns the source plate so that the source plate fits inside the alignment collar when the source plate and single unit of the device are assembled; and

wherein the cell aggregates from the wells of the source plate are collected into the collecting plate during centrifugation.

Referring to Figures 11A to 11D, an embodiment of an alignment collar 1100 and an integral collecting plate 1102 is shown. In the embodiment shown, the alignment collar 1100 is configured to fit outside a standard well plate 1104 (referred to hereinafter as the source plate 1104), such as a 96-well or 384-well plate. Collar 1100 is sealed to a base 1106 such that when the source plate 1104 is inverted and placed in the alignment collar 1100, and the combination centrifuged, the contents of the source plate 1104 are transferred into the collecting plate 1102. The source plate 1102 is shown to be supported in such a way that sufficient free volume 1108 remains under it to contain the combined well contents of the source plate 1104 without overflowing.

In another embodiment, the cell aggregates in step (3) are recovered from wells of a source plate comprising a method involving centrifugation of an assembled device, wherein the source plate is inverted; and wherein the assembled device comprises:

a) the source plate;

b) a collecting plate; and

c) a separate alignment collar;

wherein the alignment collar forms a bridge between the collecting plate and source plate; and

wherein the alignment collar fits outside the perimeter of the collecting plate and aligns the source plate, so that the source plate fits inside the alignment collar when the source plate, the collecting plate and the alignment collar of the device are assembled; and

wherein the cell aggregates from the wells of the source plate are collected into the corresponding wells of the collecting plate during centrifugation.

Referring to Figures 12A to 12D, an alternate embodiment of an alignment collar is shown. In this embodiment, the alignment collar 1200 is separate from the collecting plate 1202. In this embodiment, the alignment collar 1200 is configured to bridge the source plate 1204 and the separate collecting plate 1202, such that when the alignment collar 1200 is placed on the collecting plate 1202 (for example a 96-well plate), and the source plate 1204 is inverted and applied to the top of the alignment collar 1200, and the assembly is centrifuged, the contents of the source plate 1204 are transferred to the collecting plate 1202. The source plate 1204 is shown to be aligned such that the wells 1205 in the source plate 1204 align with the desired destination wells 1203 in the collecting plate 1202.

In either of the above embodiments, the source plate and/or collecting plate may incorporate the microwell device described above and in Figure 1.

In another embodiment, the collecting vessel may be a standard multi-well plate. For example, the standard multi-well plate may be a 6-well, 96-well or 384-well plate. In yet another embodiment, the source plate that fits inside the alignment collar is a multi-well plate and the collecting plate is a multi-well plate, such that when the source plate is inverted, the cells from the wells of the source plate are collected into the corresponding wells of the collecting plate in the assembled device. This allows the contents from separate regions of the well plate, or source plate to maintain separation when the cells in the source plate are transferred the wells of the collecting plate.

In another embodiment, it is necessary that when the device is assembled, there be sufficient unoccupied volume in the collecting vessel to contain the contents of the source plate. Optionally, in another embodiment a gasket of liquid-resistant material may be inserted in between the source and collecting plates to prevent leakage or cross-contamination between wells when both plates contain multiple wells.

In another embodiment, the assembled device is centrifuged. In yet another embodiment, the device is used to recover cell aggregates from mammalian pluripotent stem cells such as mammalian embryonic stem cells aggregates or embryoid bodies. In another embodiment, the mammalian pluripotent stem cells are human.

The "spin-out" technology method described above for recovering cells from standard format well plates permits the use of higher plate densities, and is compatible with the robotics, automation and scale-up that will be required to produce clinically useful numbers of differentiated cells via cell aggregates from mammalian pluripotent stem cells, such as mammalian embryonic stem cells.

This application also discloses improved methods for reproducibly generating large numbers of uniform and consistent aggregates of cells such as mammalian pluripotent stem cells including human embryonic stem cells and other pluripotent cells. Based on the applicants' observations, the reproducibility of forced aggregation of mammalian pluripotent stem cells, such as human embryonic stem cells is dependant on appropriate differentiation occurring, and that the level of this differentiation is controlled by factors including, but not limited to, pre-differentiation with serum; pre-differentiation with growth factors such as Bone Morphogenetic Proteins [BMPs]; the addition of cell survival factors such as an inhibitor of p160-Rho-associated coiled-coil kinase (ROCK); withdrawal of differentiation-inhibiting factors such as fibroblast growth factor [FGF], activin, and transforming growth factor-beta [TGF-beta], withdrawal of MEF cells, culture on non-supportive substrate, the addition of cells able to secrete extracellular matrix [ECM] such as MEF cells and ESC-derived differentiated cells, culture density at harvest, culture passaging density and technique.

For example, with regard to culture density at harvest, culture densities of 1 million cells per cm2 will generally make lower quality aggregates (or no aggregates at all) as compared to cells cultured at 0.2 million cells per cm2 absent compensation with other techniques such as pre-differentiation as described above. With regard to culture passaging density, generally, cultures that are passaged at a 1:6 ratio (i.e. one well of ESC is split to 6 wells for further growth) seem to perform poorly in comparison to cultures passaged at a 1:12 ratio (absent pre-differentiation etc). In addition, when pluripotent stem cells such as ESC are passaged, the colonies are generally broken up into smaller units, where the breaking up of cells into smaller clumps is expected to improve aggregate forming ability.

If cells are excessively pre-differentiated using these techniques, highly coherent aggregates are formed however they are then resistant to release from the wells in which they are aggregated, and their subsequent differentiation trajectory may also be affected. The precise degree of pre-differentiation that is optimal will vary with the cell line in question (e.g. pluripotent/embryonic stem cell lines) and exact maintenance techniques including, but not limited to, passage ratio (e.g. 1:6 vs 1:12), colony size, medium formulation employed, and the density cells are allowed to reach before passaging. If cell aggregate or EB coherence is still insufficient after treatment as above, pre-differentiation can be started earlier (i.e the duration increased) or (if using BMPs), BMP concentration can be increased. If highly coherent aggregates result but are not efficiently recovered from the plate in which they are formed, the duration of pre-differentiation can be reduced or (if using BMP2) the concentration of BMP2 may be reduced. In general, the period of pre-differentiation with a factor such as serum or a growth factor such as BMP, may occur from 24 to 120 hours. In general, the concentration of BMP used may range from 3 ng/ml to 50 ng/ml.

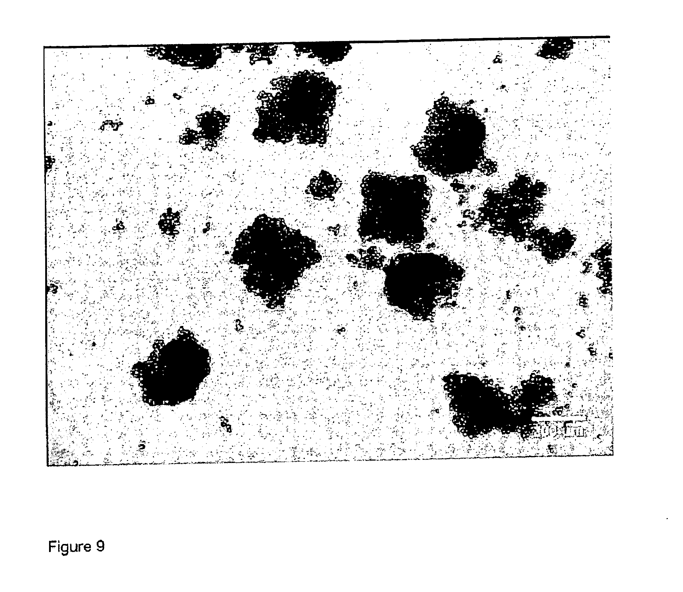

In general, the applicants' method of generating cell aggregates involves the culturing of cells, preferably pluripotent stem cells or tumor cells see Figure 9, wherein the culture may be modified by pre-differentiation, via replacing the growth medium with medium containing serum or a growth factor such as BMP, via harvesting at low confluence or by using cultures that had been passaged to low density. The cells are then harvested to obtain a suspension of cells, which are then used to make aggregates. For example, the cells may be made into a single cell suspension and then may be centrifuged into either a well plate or the device illustrated in Figure 1. The cells are then left for a period of time, usually around 16 to 48 hours, preferably 24 hours for stem cells, during which the stem cells stick together into aggregates ("first incubation"). Following this period, the aggregates may be recovered by either pipetting or by spinning out the aggregates into the device illustrated in Figures 11 and 12. Following this procedure, the aggregates may be maintained in suspension for a period ranging from 1 to 6 days ("second incubation"), preferably 2 days. The aggregates may then be harvested for analysis or further processing. Using this protocol, the applicants' method creates high quality aggregates which self-organize over time. This may occur in the original well plate during the first incubation or after recovery during the second incubation. The applicants have noted that the key parameter may be the total elapsed time since the step at which the aggregates were formed by centrifugation of the single-cell suspension in the well plate.

Accordingly and as described above, the application provides a method of generating cell aggregates from mammalian pluripotent stem cells comprising:

(1) preparing a population of undifferentiated mammalian pluripotent stem cells and differentiated cells by adding a factor to the mammalian pluripotent stem cells to promote cell differentiation in part of the population, or by separately culturing the mammalian pluripotent stem cells and differentiated cells; and

(2) preparing a mixture in suspension comprising the differentiated cells and undifferentiated cells of step (1); and

(3) forming cell aggregates from the mixture in step (2).

In one embodiment, the mammalian pluripotent stem cells are mammalian embryonic stem cells. In another embodiment, the mammalian embryonic stem cells are human embryonic stem cells.

In one aspect, the factor referred to in step (1) is serum or a growth factor, such as bone morphogenetic protein (BMP). In one embodiment, the factor described in step (1) is added to the mammalian pluripotent stem cells for a period of 24 to 120 hours. In another embodiment, the period is 48 hours. In another embodiment, BMP-2 and BMP-4 may be used as the BMP. In another embodiment, the concentration of BMP used may range from 3 ng/ml to 50 ng/ml. In a further embodiment, the concentration of BMP-2 used is 25 ng/ml. In yet a further embodiment, the concentration of BMP-4 used is 50 ng/ml BMP4. Other factors include but are not limited to withdrawal of differentiation-inhibiting factors such as FGF, activin, TGF-beta. In one embodiment, the serum is fetal bovine serum [FBS]. In another embodiment, the serum added is a differentiation medium, which consists of KO-DMEM, fetal bovine serum [FBS], glutamax-I, MEM non-essential amino acids and penicillin/streptomycin. In another embodiment, the differentiation medium consists of KO-DMEM, 15% FBS, 1% Glutamax-I, 1% MEM non-Essential Amino Acids, and 1% Penicillin/Streptomycin.

In another aspect, a separate population of differentiated cells is added to the stem cells. In one embodiment, the differentiated cells are mouse embryonic fibroblasts. In another embodiment, the differentiated cells are derived from the mammalian pluripotent stem cells. In general, the addition of differentiated cells facilitates formation of the cell aggregates, likely by secreting ECM and/or pro-survival factors.

In a further aspect, a promoter of cell survival may be added to the stem cells in order to enhance aggregate formation, with or without pre-differentiation or the addition of differentiated cells. In one embodiment, the promoter of cell survival is a selective inhibitor of p160-Rho-associated coiled-coil kinase (ROCK). In a specific embodiment, the ROCK inhibitor is Y-27632.

In one embodiment, the population in step (1) is passaged at a low cell density. In another embodiment, the low cell density in the ranges from of a 1:7 to 1:12 dilution of the density prior to passaging. In a further embodiment, the low cell density is a 1:12 dilution of the density prior to passaging.

In another embodiment, the population in step (1) is harvested at a low confluence level. In another embodiment, the low confluence level is less than 0.5 million cells per cm2. In another embodiment, the low confluence level is 0.2 million cells per cm2, which is equivalent to approximately 20%. In another embodiment, the low confluence level ranges from 5% to 50%. In another embodiment, the cells are harvested at 20% confluence. In another embodiment, the cell aggregates may be separated from unincorporated cells and debris after harvesting. In another embodiment, the cell aggregates may be separated from unincorporated cells and debris after harvesting by dispensing the suspension over a filter. In another embodiment, the filter is a 40 micron filter.

In one embodiment, the mixture of cells in step (2) is dispensed in low-adsorbance plates, for example 96 well or 386 well plates, or into the device described above and in Figure 1. In another embodiment, the mixture in step (2) is centrifuged to form the aggregates in step (3). In another embodiment, the mixture in step (2) is centrifuged into low-adsorbance plates. In another embodiment, low-adsorbance plates may be made by coating well plates with pluronic acid. In yet another embodiment, commercially available ultra-low adsorbance plates may be used. In a further embodiment, the mixture in step (2) is centrifuged into the microwell device illustrated in Figure 1, which is used to form the aggregates. In another embodiment, the cell aggregates are recovered using the device shown in Figure 11 or Figure 12 as discussed above. As mentioned previously, the microwell device of Figure 1 may be used as the collecting plate in the embodiments described in Figures 11 and 12.

This application further comprises a method whereby consistent cell aggregates of mammalian pluripotent stem cells, such as embryonic stem cells aggregates or EBs, formed from appropriate numbers of cells are able to self-organize to form tissue-level structure around a single organizing center. This method addresses the need in the art for efficient production of tissue-level order within cell aggregates such as embryonic stem cell aggregates or EBs. This method comprising the method set out above and furthering comprises an additional step of maintaining the recovered cell aggregates in suspension or in the wells or microwells for an extended period, referred to above as the "second incubation" wherein the resulting cell aggregates exhibit tissue level organization within the cell aggregates. In one embodiment, the extended period is 2 to 120 hours. In another embodiment, the extended period is up to 7 or 8 days or greater. In another embodiment, the period is 24 hours. In another embodiment the mixture of cells in step (2) comprises 2 to 100,000 cells. In a further embodiment, the mixture of cells in step (2) is 2000. The method described substantially reduces the chaos and disorder characteristic of existent protocols and results in high quality cell aggregates that exhibit tissue level organization within the embryoid bodies. In one embodiment, the cell aggregates may self-organize over time. In one embodiment, this may occur during the first incubation, or after recovery in the second incubation. In another embodiment, the key parameter is the total elapsed time since the step at which the aggregates were formed by centrifugation of the single-cell suspension in the well plate.

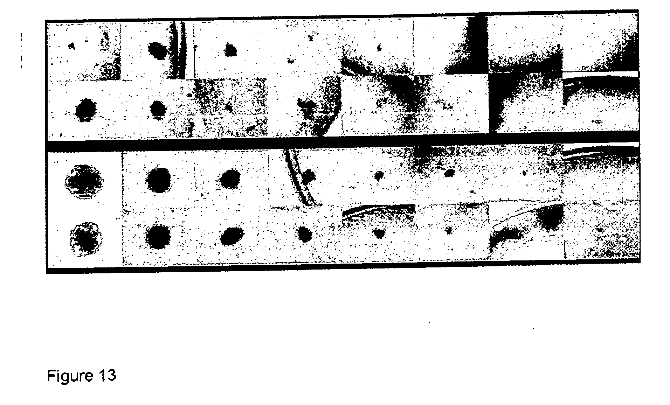

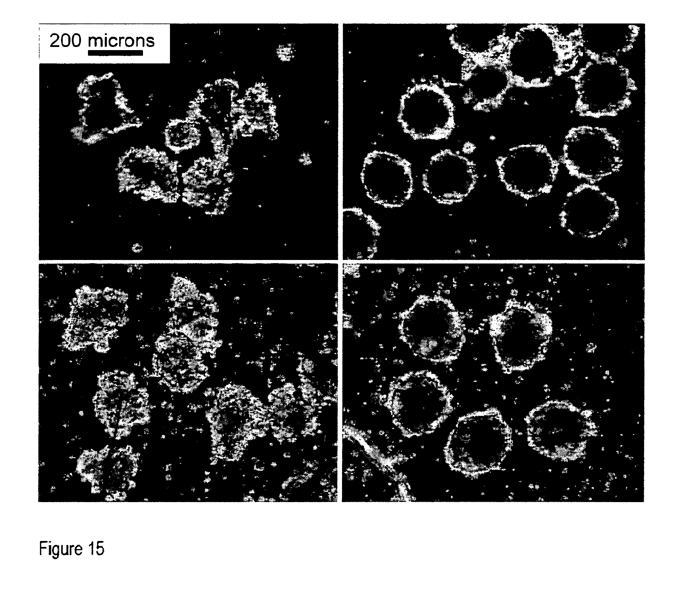

In one embodiment, visualization of the tissue level organization is by confocal microscopy. In another embodiment, visualization may occur via light microscopy, optical coherence tomography, or high-resolution ultrasound. In another embodiment, tissue level organization is visualized by assessing expression of marker proteins, such as E-cadherin and Oct4, and by assessing structural organization, such as columnar morphology and actin cytoskeleton. In one embodiment, actin cytoskeleton may be probed with phalloidin. In another embodiment, marker proteins such as GATA6 and Laminin may be assessed. In a further embodiment, FoxA2 and beta-catenin may be assessed. In another embodiment, structural organization may be observed by cavitation, epithelialization, polarization and segregation. For an example of polarization, see Figure 15, where the Oct4-labelled nuclei (red) are generally at the ends of the cells nearest the interface with the GATA6-labelled cells (green). For an example of segregation, see Figures 13 to 15 - in the most general case segregation is any organized or partially organized arrangement of more than one cell.

In one embodiment, centrufigation in the spin out step occurs at a range of 2 x g to 1000 x g. In another embodiment, centrifugation occurs at 16 x g to 200 x g. In another embodiment, centrifugation occurs at 20 x g. In another embodiment, the assembled device is centrifuged for 20 seconds to 5 minutes. In yet another embodiment, the assembled device is centrifuged for 1 minute. In yet another embodiment, the method is used to recover cell aggregates from mammalian pluripotent stem cells such as mammalian embryonic stem cells aggregates or embryoid bodies. In another embodiment, the mammalian pluripotent stem cells are human. In another embodiment the mammalian pluripotent stem cells are differentiated to a specific fate via the addition of exogenous factors. In another embodiment the exogenous factors and culture medium are chemically defined. In another embodiment, greater than 10,000 aggregates are generated for subsequent use. In another embodiment, greater than 100,000 aggregates are generated for subsequent use. In another embodiment the subsequent use includes transferral to a stirred suspension bioreactor cultivation system.

The following non-limiting examples are illustrative of the present invention:

Examples

Example 1: Use of microwells for the generation of hESC aggregates.

A silicon master mould was generated via KOH anisotropic etching techniques as described previously, see Figure 2 left column, and PDMS replica moulding was employed to generate a tiled array of microwells in PDMS as described above (Paragraphs 134-137), see Figure 2 center and right columns. Sections of the arrays of 800 and 200 micron PDMS microwells were cut manually to size with a razor blade, and transferred into individual wells in a 96-well plate. A single-cell suspension of hESC cultured on Matrigel was then centrifuged onto the surfaces in the presence of 10 µM of the ROCK inhibitor Y-27632, and incubated overnight. The following day, the resulting aggregates were recovered by manual pipetting (see Figure 3). As the PDMS microwell arrays were cut to size manually for this prototype experiment, coverage of the well bottom was imperfect, resulting in the formation of some randomly-sized aggregates from cells that did not fall into a covered region, but instead collected in the base of the well. It is apparent from Figure 3C however that several hundred uniform aggregates were generated from 200 micron microwells within a single well of the 96-well plate, representing an improvement in yield of over two orders of magnitude over the current state of the art, where a single aggregate is generated per well.

Example 2: Generation of uniform hESC aggregates without centrifugation.

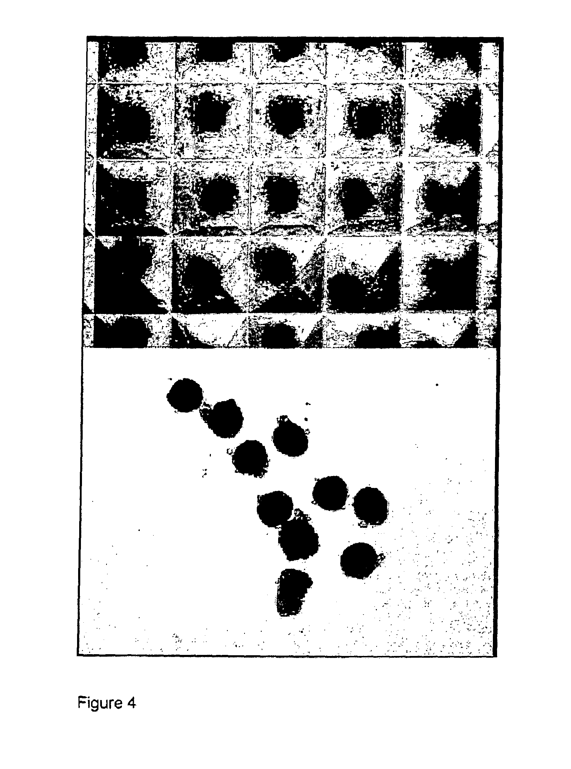

hESC cultured on Matrigel were treated with 10 µM Y-27632 and allowed to settle into 200 micron PDMS microwells in the device schematized in Figures 1A-F and depicted Figure 2 without further centrifugation, and aggregate for 24 hours. Figure 4 upper panel shows the aggregates in the microwells, lower panel shows aggregates after extraction.

Example 3: Use of microwells as a culture surface.

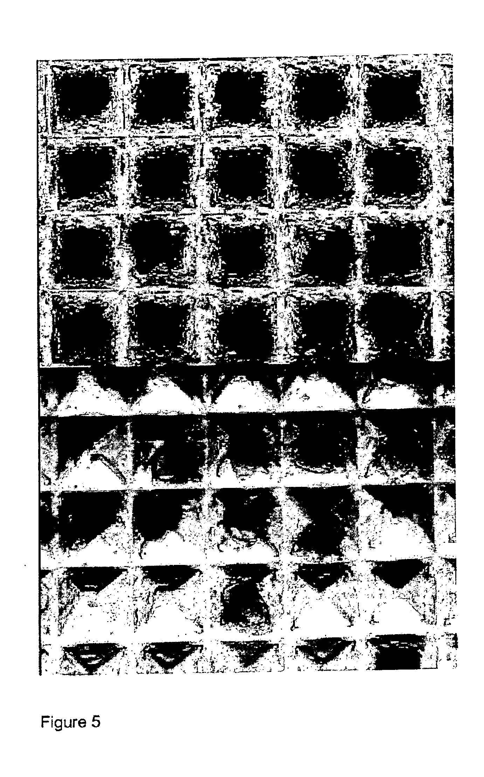

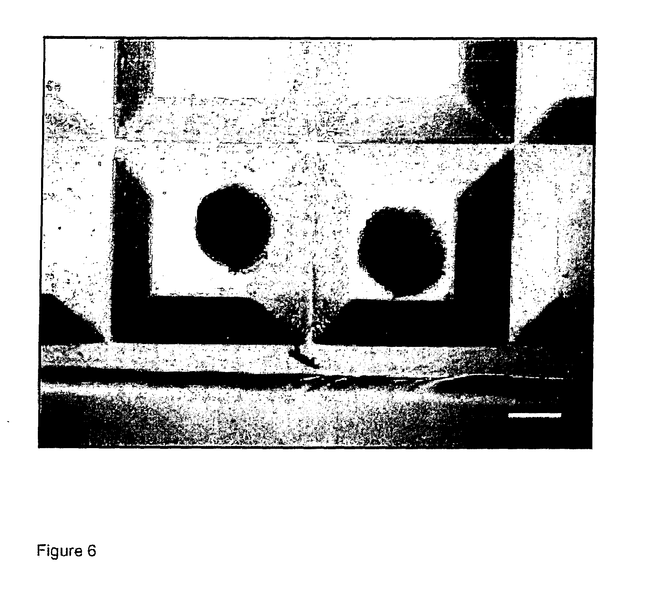

hESC cultured on MEF were pre-treated with serum containing medium for 48 hours, and centrifuged into 200 micron PDMS microwells. Figure 5 upper panel shows the aggregates in the microwells after 24 hours. A portion of the aggregates were extracted, the remainder were refed in situ, lower panel shows aggregate development after an additional 48 hours in the wells. As shown in Figure 6, objects prepared using any technique (in this case forced aggregation of hESC in a 96-well plate format) may be transferred onto microwell surfaces for culturing, facilitating observation and refeeding and inhibiting interactions between objects.

Example 4: Use of microwells is not limited to hESC aggregates.

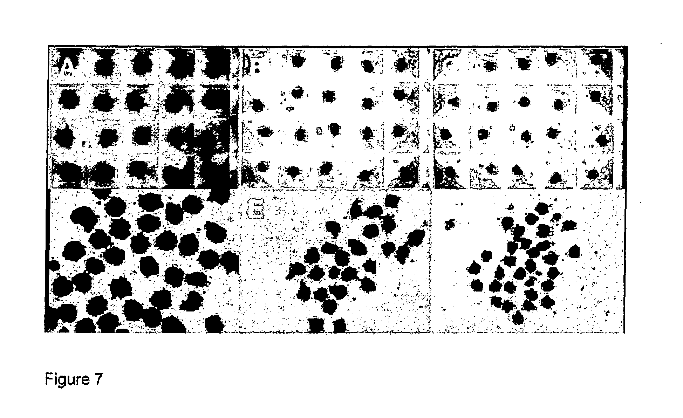

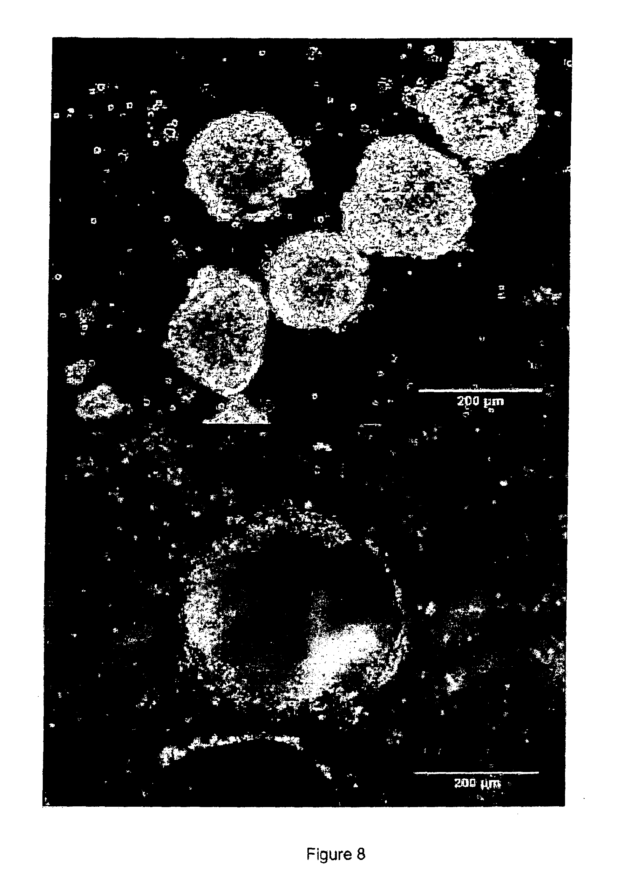

Mouse embryonic fibroblasts cells were loaded as a single-cell suspension into 400 µm wells at 2,000 (A,D), 1,000 (B,E) or 500 (C,F) cells per microwell and centrifuged. Figure 7 panels A through C show the aggregates in the microwells after 24 hours, panels D through F show the aggregates after subsequent extraction. Figure 8 upper panel shows aggregates prepared from a mouse ESC line in the microwell system. The cell line employed in this case expresses the Green Fluorescent Protein (GFP) as a reporter under the control of the Brachyury promoter, whose expression was detected after 6 days of culture (Figure 8 lower panel).

Example 5. The use of microwells for preparation of aggregates of tumor cells ("tumor spheroids"). HeLa tumor cells were aggregated in the device at 1,000 cells per microwell, incubated for 24 hours at 37 degrees / 5% CO2, extracted and imaged (Figure 9).

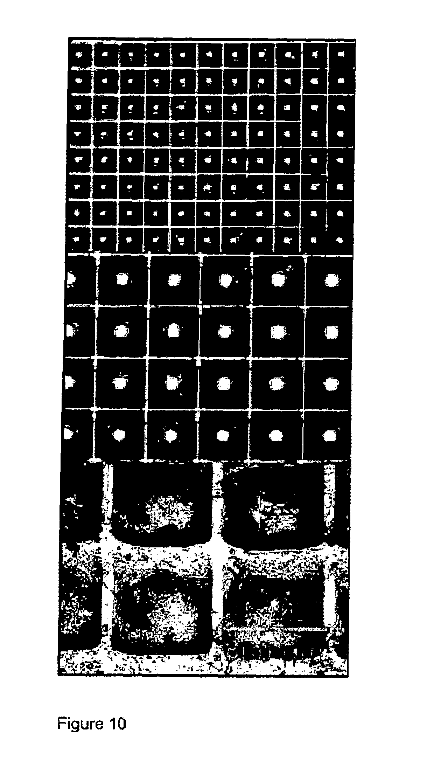

Example 6. Positive and negative microwells can be generated in differing materials, Figure 10 shows a negative image (micropyramids) of 200 µm microwells in high temperature epoxy (top); a negative image (micropyramids) of 400 µm microwells in epoxy; and 800 µm microwells hot-embossed directly into the plastic in the culture surface of a standard 6-well tissue culture plate using techniques derived from Koerner et al., 2005.

Example 7: Method of reproducibly generating large numbers of uniform and consistent aggregates of human embryonic stem cells

Materials:

□ hESC in culture

□ TrypIE Express (Invitrogen cat# 12605-028)

□ 96-well V-bottom plates (Corning Costar cat# 3896) or 384-well V-bottom plates (Whatman cat#7701-5101) with lids (Whatman cat#7704-1001)

□ Pluronic F-127 (Sigma cat #P2443-1 KG)

□ Dulbecco's Phosphate Buffered Saline (DPBS - Invitrogen cat# 14190-250)

□ Alignment collar with integral or separate collecting plate (see Figures 1 and 2, respectively) or large-bore pipette tips (for 96-well plates - Molecular BioProducts cat# 3531) or standard pipette tips (Sarstedt cat# 70.760.502) with the ends cut off to enlarge the bore

□ X-Vivo10 (Cambrex, cat# 04-380Q)

□ Differentiation medium [DM - KO-DMEM (Invitrogen, cat# 10829018) + 15% FBS (Hyclone, cat #SH30088.03) + 1% Glutamax-I (Invitrogen cat#35050061) + 1 % MEM Non-Essential Amino Acids (Invitrogen cat# 11140050) + 1% Penicillin/Streptomycin (Invitrogen, cat# 15140-122)] or BMP2 (R&D Systems, cat#355-BM-010)

□ Optional: 40 micron filter unit (BD Falcon cat# 352340)

Protocol:

1. Human Embryonic Stem Cells (hESC) cultured using standard techniques do not consistently form high-quality aggregates using the "spin-EB" protocol as published (Ng_2005;Burridge_2007) (aggregates are often loose, poorly defined and/or cannot be recovered intact). The applicants have determined several ways in which this problem can be resolved (alone or in combination):

a) for 48 hours prior to harvesting, replace growth medium with DM (above); or

b) for 48 hours prior to harvesting, replace growth medium with X-Vivo10 + 25 ng / mL BMP2; or

c) when passaging cells, keep initial colony sizes small and consistent, decrease initial seeding density, and harvest the culture well before confluency is reached or

d) form aggregates in the presence of 10 µM Y27632

If cells are excessively pre-differentiated using techniques a-c, highly coherent aggregates are formed however they are then resistant to release from the wells in which they are aggregated, and their subsequent differentiation trajectory may also be affected. The precise degree of pre-differentiation that is optimal will vary with the cell line in question, exact maintenance techniques, etc. If EB coherence is still insufficient after treatment as above, pre-differentiation can be started earlier (i.e the duration increased) or (if using BMPs), BMP concentration can be increased. If highly coherent aggregates result but are not efficiently recovered from the plate in which they are formed, the duration of pre-differentiation can be reduced, or (if using BMP2) the concentration of BMP2 can be reduced.

2. Prepare low-adsorbance well plates:

a) dispense 40 µL (for 96-well plates) or 25 µL (for 384-well plates) of 5% Pluronic F-127 in PBS into the plates

b) incubate 30 minutes at room temperature

c) remove Pluronic F-127 solution by pipetting or by inverted centrifugation in the device described and illustrated in Figures 1 or 2 for 1 minute at 240 x g

3. Prepare a single-cell suspension by treating the hESC culture with TrypIE for 5 minutes, and washing the cells off with X-Vivo10

4. Count cells, spin down and re-suspend to 1 million cells / mL in X-Vivo or other medium of interest. Cells may all derive from a single culture, or cells from different sources may be combined.

5. Suspend cells in X-vivo10 or other medium of interest at a concentration appropriate to the desired EB size and plate format (e.g. to form aggregates from 10,000 cells in 100 µL medium each in 96-well plates, suspend 1 million cells in 10 mL; for aggregates from 2,000 cells in 25 µL medium each in 384-well plates, suspend 0.8 million cells in 10 mL. NB multichannel pipettes or automated liquid handling systems often require a minimum "dead volume", thus it may be necessary to make 10-20% additional volume of suspension depending on the specifics of the equipment employed.

6. Dispense the cell suspension into the well plates prepared in step 2.

7. Centrifuge for 5 minutes at 240 x g

8. Incubate overnight at 37 degrees, 5% CO2 in a standard tissue culture incubator.

9. Recover EBs by pipetting using large-bore or cut-off pipette tips or by inverted centrifugation in the device described and illustrated in Figures 1 or 2 for 1 minute at 20 x g

10. Optional: EBs may be separated from unincorporated cells and debris, and the medium changed by dispensing the EB suspension over a 40 micron filter. The filter is then inverted, and the EBs washed off into the new growth medium.

Example 8: Method to produce EBs which are able to self-organize to form tissue-level structure around a single organizing center.

Materials:

□ 6- or 96-well ultra-low-attachment plates (Corning Costar cat# 3471 and 3474 respectively)

Protocol:

1. Generate aggregates of 2,000 cells each using e.g. the protocol described above as Example 7.

2. Maintain aggregates in suspension without inter-aggregate aggregation for an additional 24-120 hours (for example in individual wells in 96-well plates or at low aggregate densities in 6-well plates [e.g. aggregates formed in a single 384-well plate distributed between 6 or more wells in a 6-well plate format]) at 37 degrees / 5% CO2 in a standard tissue-culture incubator.

Example 9: Pre-differentiation of hESC cultures with serum is sufficient to permit stable aggregate formation from cultures that would not otherwise do so.

Aggregates were formed using the protocol described as Example 7, Protocol Steps 1-9, from 100 µL of a suspension containing serial 2-fold dilutions of cells, starting with 100,000 cells per aggregate (see Figure 13). As shown in Figure 13, successive columns show aggregates formed from equal volumes of serial 2-fold dilutions of the cell suspension, starting with 100,000 cells (left-most column). Paired rows represent duplicates. Aggregates formed reliably from cells that were pre-differentiated in serum-containing medium or differentiation medium [DM], while this was not the case with the control cells. Aggregates that did form from the control population were smaller and/or less cohesive.

Example 10: Cells harvested at lower densities produce more coherent aggregates than cells harvested at high densities.

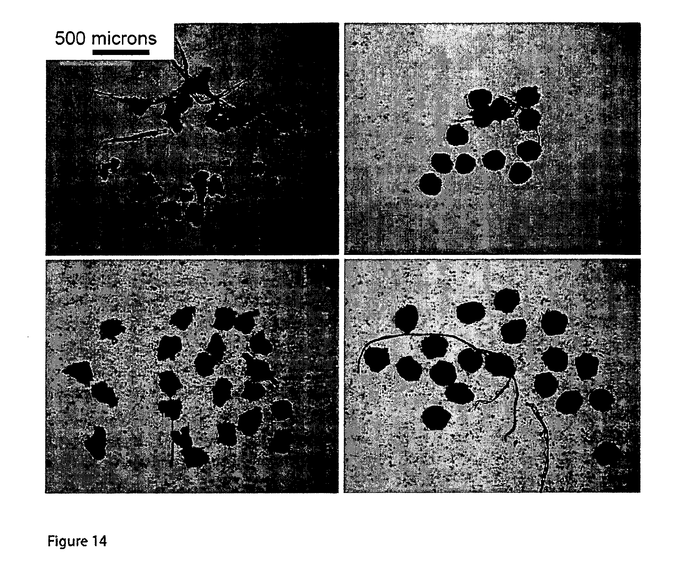

Aggregates were formed using the protocol described as Example 7, from hESC cultured on mouse embryonic fibroblasts (MEF) to 20% or 90% confluence. Aggregates formed from cells harvested at lower confluence level were more coherent and regular (see Figures 14 and 15, left columns, compare upper and lower rows).

Example 11: Supplementation with MEF cells increases aggregate stability.

Aggregates were formed using the protocol described as Example 7, Protocol Steps 1-9, from hESC cultured on MEF to 20% or 90% confluence, either as harvested, or supplemented with additional MEF cells. Aggregates formed from cells supplemented with additional MEF cells were more coherent and regular (see Figures 14 and 15, compare left with right columns).

Example 12: Aggregates are able to differentiate to endodermal, ectodermal and mesodermal lineages including cardiac, haematopoetic and neural cell types.

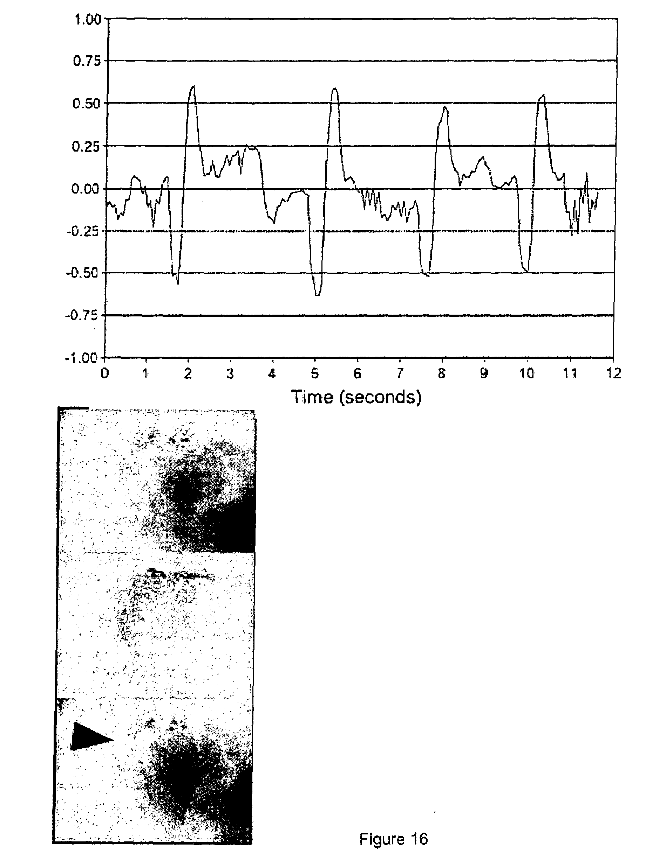

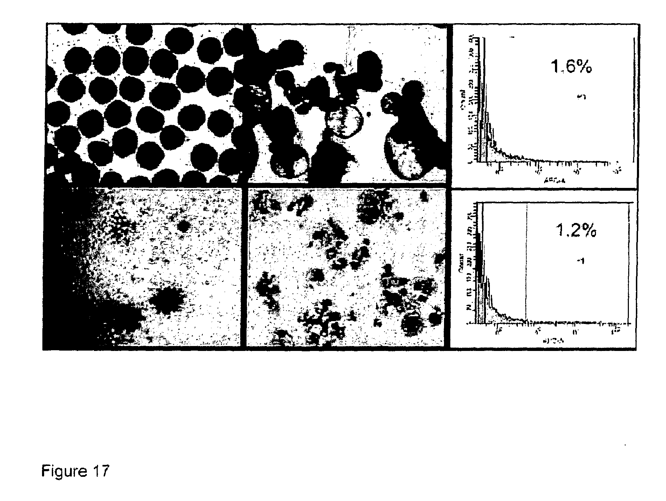

Aggregates were formed using the protocol described as Example 7, Protocol Steps 1-9, from hESC cultured on MEF. Aggregates maintained under conditions known to promote cardiac differentiation in EBs gave rise to rhythmically beating structures (see Figure 16), while under conditions known to promote haematopoesis in EBs, haematopoetic cells were detected via several different techniques (see Figures 17, 27). Neural differentiation was also observed via the formation of neural rosette structures staining positive for Pax6 and Sox2 (Figure 27. Quantitative RT-PCR results from aggregates formed from hESC cultured on MEF differentiated for 4 days in suspension followed by an additional 3 days in adherent culture shows down-regulation of pluripotency genes, and up-regulation of markers for endodermal, ectodermal and mesodermal lineages (see Figure 27).

Example 13: Pre-differentiation of hESC cultures with BMP2 is sufficient to permit stable aggregate formation from cultures that would not otherwise do so.

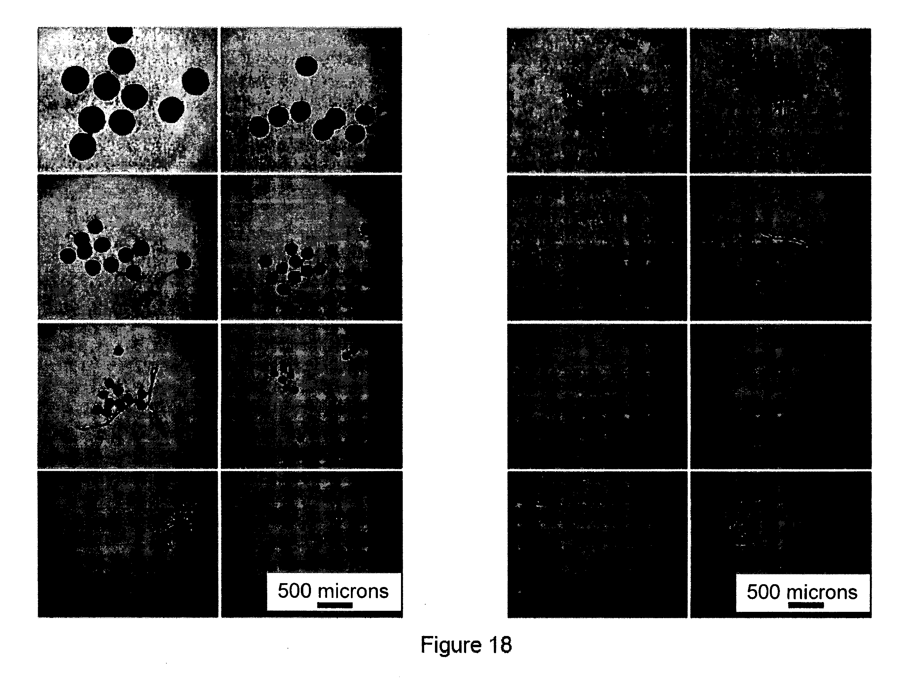

Aggregates were formed using the protocol described as Example 7, Protocol Steps 1-9, from hESC cultured on Matrigel with (Figure 18, left side) or without (Figure 18, right side) BMP2 pre-differentiation. Aggregates formed from the pre-differentiated population, while they did not form from the control population. BMP-based pre-differentiation is significant in that it represents a potentially xeno-free means of preparing hESC for aggregate formation, an important consideration for future protocols intended for clinical applications.

Example 14: Pre-differentiation of hESC cultures with BMP4 is sufficient to permit stable aggregate formation from cultures that would not otherwise do so.

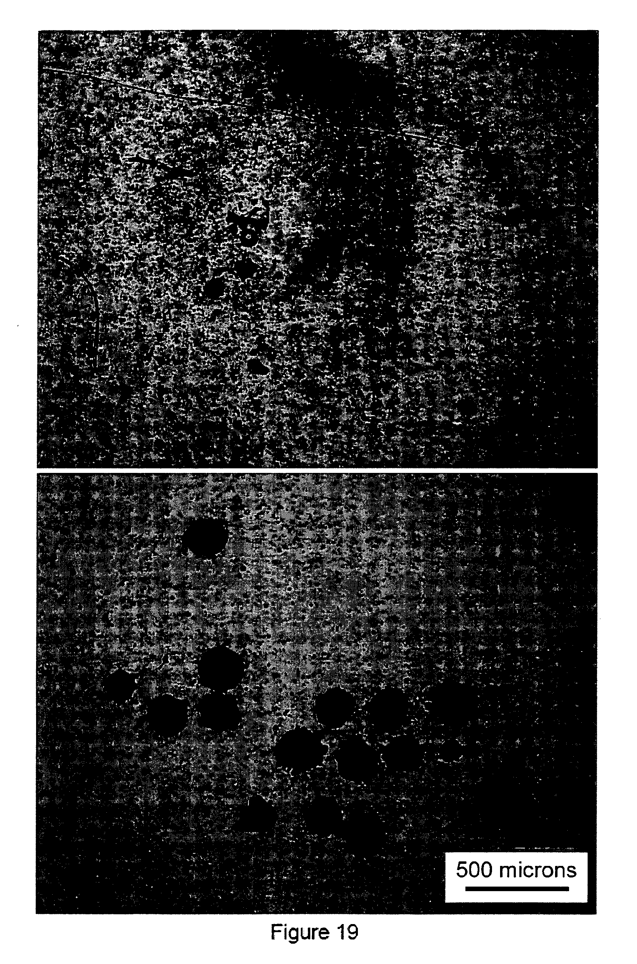

Aggregates were formed using the protocol described as Example 7, Protocol Steps 1-9, from hESC cultured on Matrigel without (Figure 19, top panel) or with (Figure 19, bottom panel) BMP4 pre-differentiation. Aggregates formed from the pre-differentiated population, while they did not form from the control population.

Example 15: Appropriately sized aggregates self-organize into an ordered and a disordered domain.

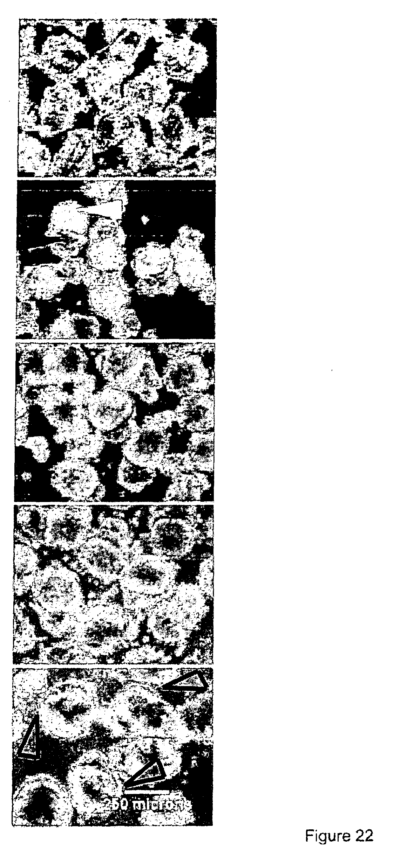

Aggregates were formed from 2,000 cells each, using the protocol described as Example 7, from hESC cultured on MEF. They were then transferred to ultra-low-adsorbance cultureware, and allowed to self organize for 0, 1, 2, 3, or 4 days (see Figure 22). The aggregates exhibited rapid self-organization into an ordered domain (minimally light-scattering) and a disordered domain (highly light-scattering). The consistency and reliability of this self-organization depends necessarily on the consistency and reliability of the initial aggregates.

Example 16: Self-organized aggregates exhibit characteristics of organized epiblast and extraembryonic endodermal tissues.

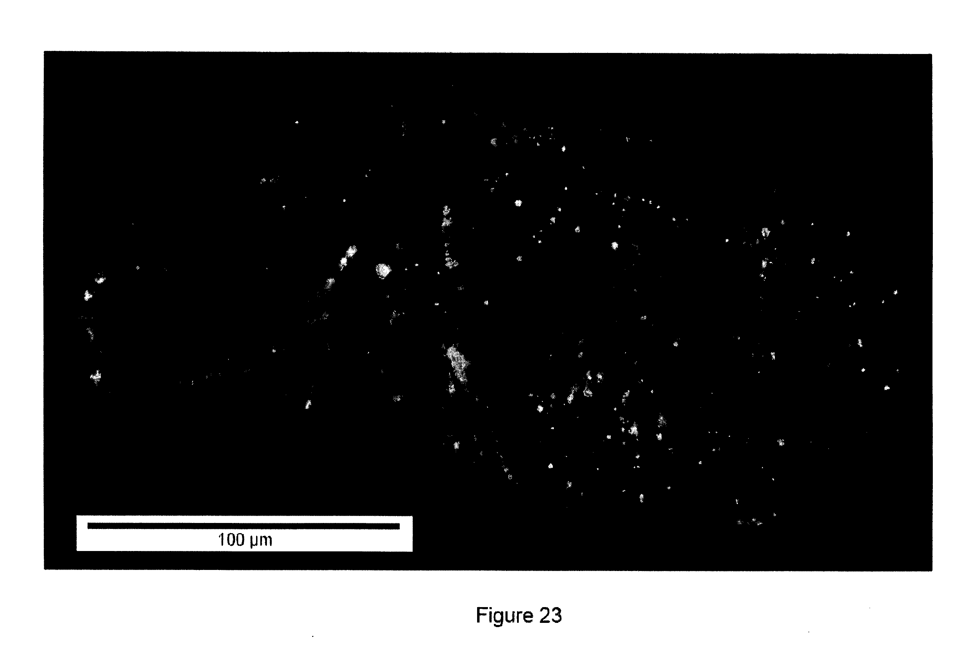

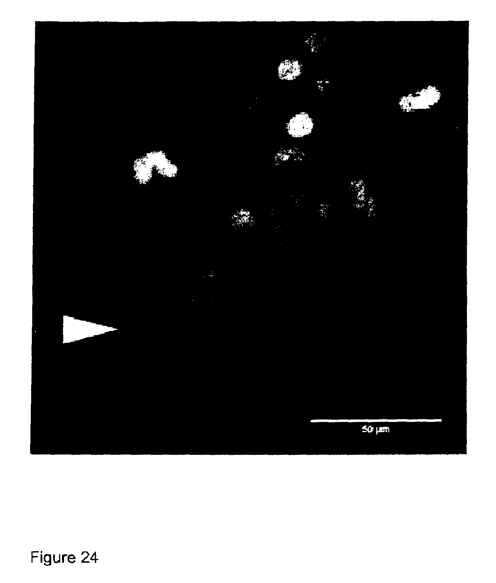

Aggregates were formed from 2,000 cells each, using the protocol described as Example 7, from hESC cultured on MEF. After the aggregates were allowed to self-organize, confocal immunofluorescence microscopy revealed distinct organization of the two aggregate domains, both in terms of marker proteins expressed, and structural organization (see Figures 23 and 24). The ability to reliably generate organized tissue from human embryonic stem cells represents a significant advance in the state of the art.

Example 17: Altering the ratio of differentiated to undifferentiated cells in the input population can be used to modify aggregate behaviour.

Aggregates were formed from 2,000 cells each, using the protocol described as Example 7, from hESC cultured on MEF. Aggregates were formed either from the hESC cultures alone, or from equal numbers of cultured hESC, and hESC-derived cells differentiated with BMP2. Significant structural changes were observed in the disordered domains of aggregates formed with the addition of BMP2-differentiated cells (see Figure 25).

Example 18: Aggregates are easily recovered using the spin-out technique.

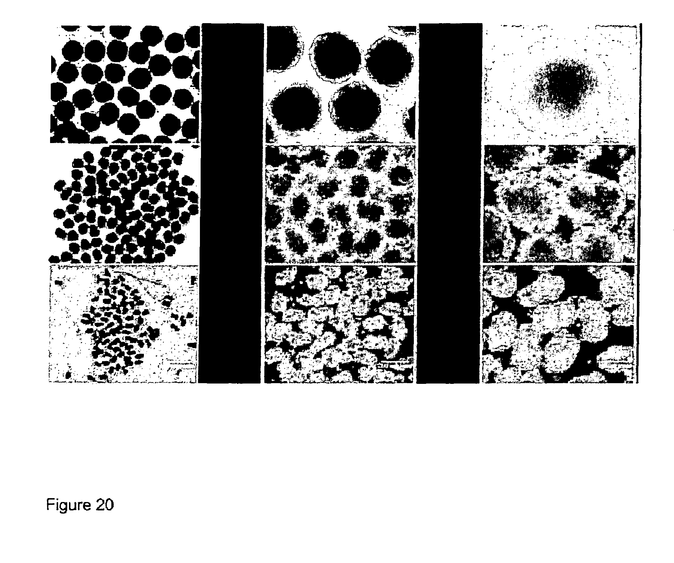

Aggregates were formed using the protocol described as Example 7, from hESC cultured on MEF, in 384-well plates, and were subsequently recovered using the inverted centrifugation technique illustrated in Figure 11, and described in paragraphs 171-177 (see also Figure 20). The ability to recover aggregates from multiple plates simultaneously in a one-minute centrifugation step represents a substantial reduction in both labour and time (recovery of aggregates from four 384-well plates takes approximately 5 minutes, including assembly of four collecting plates, a one-minute centrifugation, and transfer of the collected suspension into the desired culture vessels - as opposed to approximately 15 minutes per plate using an electronic 12-channel micro-pipette, and even longer using manual micro-pipettes). The collecting plates are re-useable and contain no moving parts, and thus also represent significant cost savings over electronic or manual multi-channel micro-pipettes.

Example 19: Aggregates are easily transferred from one plate to another using the spin-out technique.

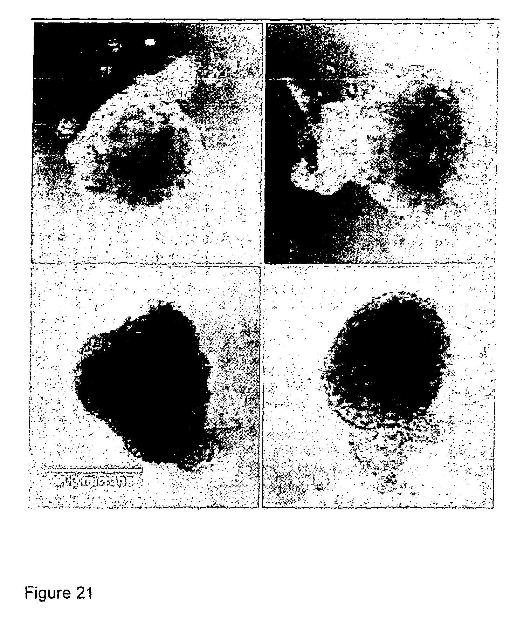

Aggregates were transferred from one 96-well plate to another using the "spin-out" technology device described above and illustrated in Figure 12 (see also Figure 21). This approach allows the inverted centrifugation technique to be employed in situations where it is desirable to maintain separation between regions in the source plate, for example when a single source plate contains aggregates formed under multiple distinct conditions.

Example 20: Use of the ROCK inhibitor Y-27632 to enhance aggregate formation.

hESC were cultured on Matrigel in conditioned medium or with 48 hours of pre-differentiation in serum-containing medium, and were subjected to the forced aggregation protocol at 2,000 cells per well with or without addition of 10 µM of the ROCK inhibitor Y-27632. As shown in Figure 26, in the absence of both pre-differentiation and Y-27632, no aggregates were formed (A). With pre-differentiation, consistent aggregates were formed (B). In both cases, the addition of Y-27632 substantially increased aggregate size and symmetry (C, D).

Example 21: Factors that control aggregate formation and stability: Pre-differentiation improves aggregate formation.

hESC cultured on mouse embryonic fibroblast (MEF) feeders were pre-differentiated with 20% serum for 72 hours prior to aggregate formation, resulting an overall reduction in population Oct4 expression (Figure 28A. right panel, red line: standard maintenance culture; green line: pre-differentiated; black line: control (unstained) population. Aggregates formed from 2000 input cells were substantially larger with treatment (green bar) than without (red bar). Y axis represents aggregate cross-sectional area in microns², error bars represent one standard deviation.

Example 22: The ROCK inhibitor Y-27632 promotes aggregate stability.

hESC cells cultured on Matrigel in MEF-conditioned medium with and without pre-differentiation were used to form SISO-aggregates in the presence or absence of 10 µM Y-27632 (Figure 28B). In the absence of both, no aggregates were formed (N.D. - size not determined). With 48 hours predifferentiation in 20% serum, consistent aggregates were formed (green bar). When Y-27632 was added to the suspension of cells without (brown bar) or with (dark green bar) pre-differentiation immediately prior to dispensing into the well plate, sizeable aggregates resulted.

Example 23. SISO-aggregation allows for the generation of size-controlled aggregates.

hEB were generated by scraping, and SISO-aggregates were generated from input populations of 400, 2,000 and 10,000 cells in 384-well plates, and recovered by centrifugation (Figure 29A). After imaging in phase-contrast mode, images were thresholded and cross-sectional areas were calculated using the ImageJ software package. Values obtained were extremely consistent, with coefficients of variation of 0.09, 0.06 and 0.08 respectively, vs 0.72 for the scraped hEB. The base-10 logarithm of cross sectional area is plotted on a histogram (Figure 29B), demonstrating the clear separation between aggregate sizes and dramatic increase in size control over scraping techniques.

### Example 24: Microwells juxtaposed with mesh or other porous structures

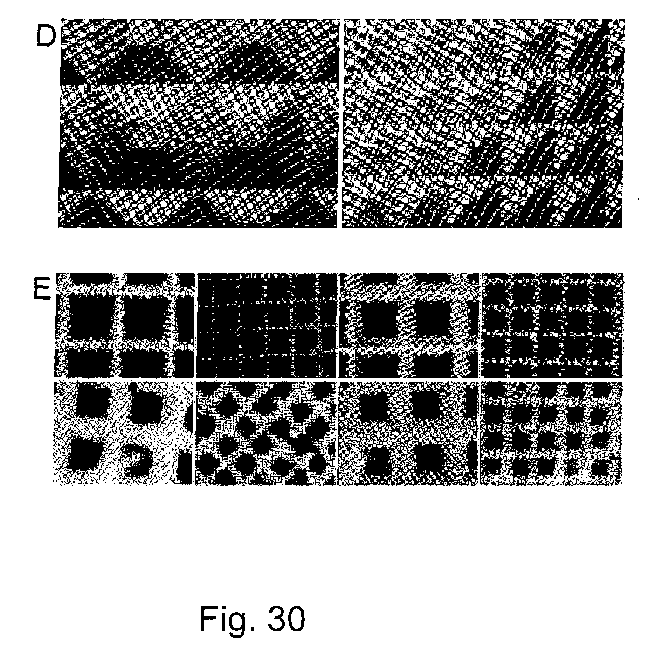

Aggregates were formed using the protocol described as Example 7 however the cells were dispensed in microwells which have a mesh juxtaposed to the microwells as shown in Figure 30. In this experiment the mesh was held in place by putting a disc of mesh on top of the microwells in a 24-well plate, and then wedging a slice of tubing into the well such that it held the edges of the mesh down against the microwells (only the lumen of the tube was usable, the microwells under the tubing wall were not accessible)

The mesh allows small objects (e.g. cells or small cell clumps) to enter the microwells but larger objects (e.g. larger cell clumps) are trapped within it. Panel (D) shows 400 and 800 micron microwells, juxtaposed to a mesh with 37-micron openings. Panel (E) shows the same device after introduction of cells (8,000, 2,000, 4,000 and 1,000 per microwell, respectively), immediately after introduction (upper row) and 24 hours later after the cells have formed aggregates (lower row).

Example 25: 200 micron microwells with 37 micron mesh glued on with PDMS

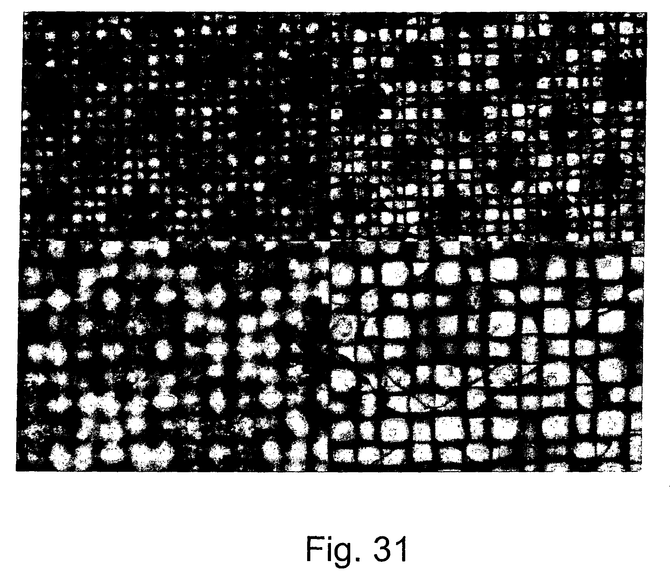

Aggregates were formed using the protocol described as Example 7 however 30 cells were dispensed in each microwell containing the 37 micron mesh as in Example 24 (Figure 31). Figure 31 shows cell aggregates formed within microwells from approximately 30 cells each. Left and right panels show same fields of view, focused on cells (left) or mesh (right).

Example 26: 800 micron polystyrene microwells containing heat welded #200 stainless steel mesh.

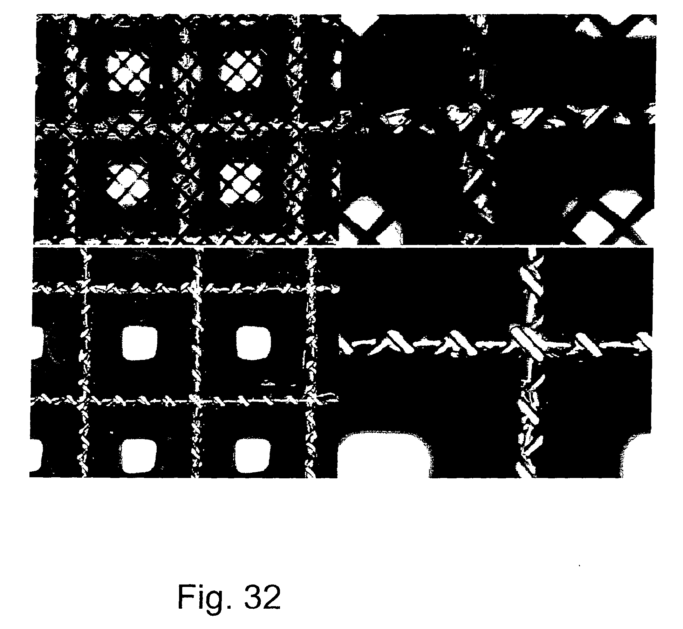

Figure 32 shows the #200 stainless steel mesh which has been heat welded to 800 micron polystyrene microwells and the same microwells after removal of the steel mesh

Example 27: 50 micron nylon mesh, heat welded to 800 micron polystyrene microwells.

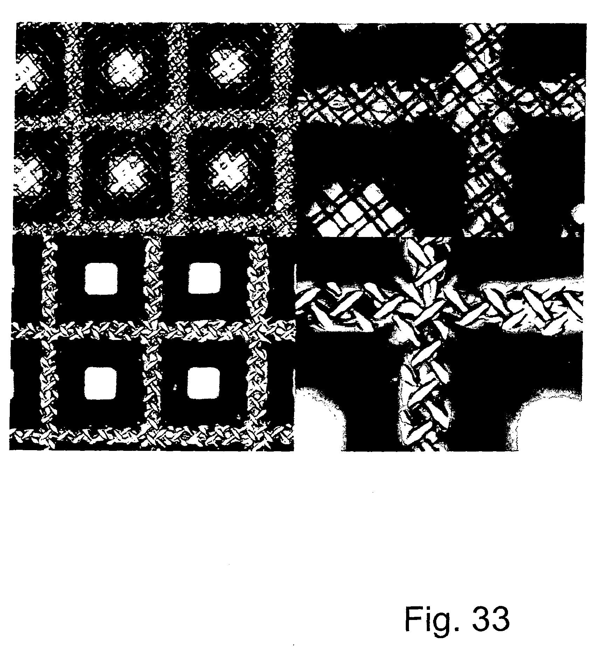

Figure 33 shows the 50 micron nylon mesh which has been heat welded to 800 micron polystyrene microwells and the same microwells after removal of the nylon mesh.

Example 28: Microwells in containers.

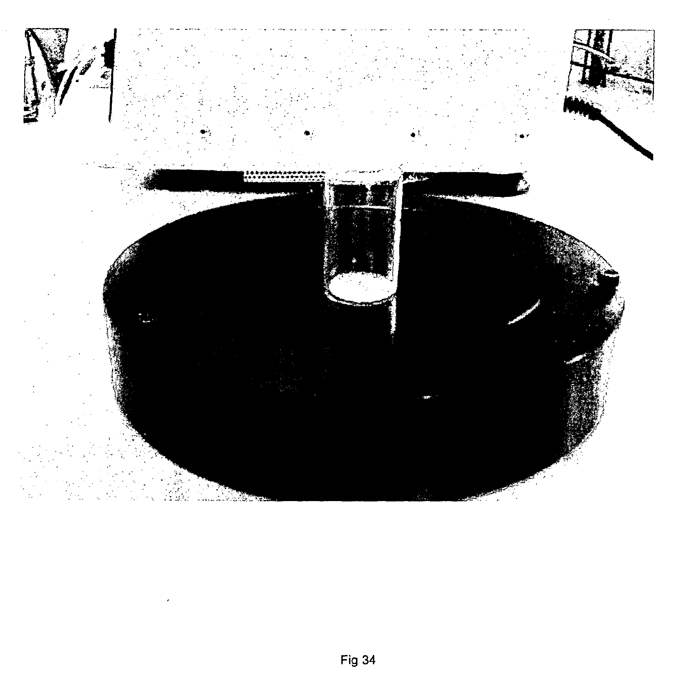

Figure 34 shows a disc of microwell-containing silicone rubber inserted into a standard glass scintillation vial.

Example 29: Microwells in containers allowing use in existing centrifuge equipment.

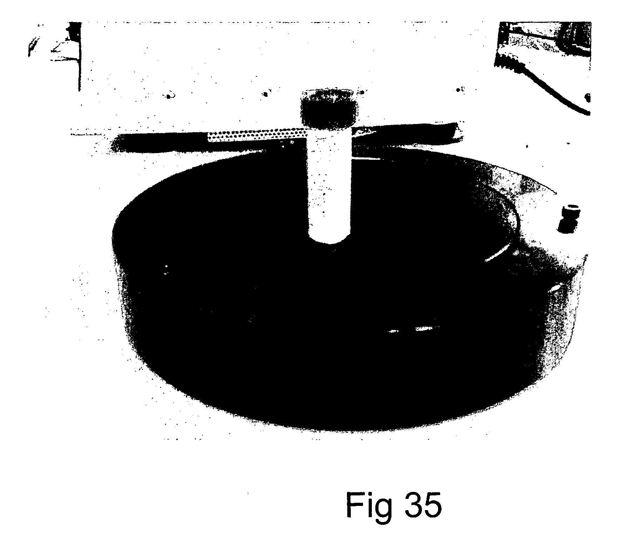

Figure 35 shows a disc of microwell-containing silicone rubber has been inserted into a modified 15 mL centrifuge tube. The end of the tube was removed, and a disc of plastic was heat-welded in its place to make a flat bottom. This device is thus suited to centrifugation in commonly available centrifuge rotors.

Example 30: Microwells in containers.

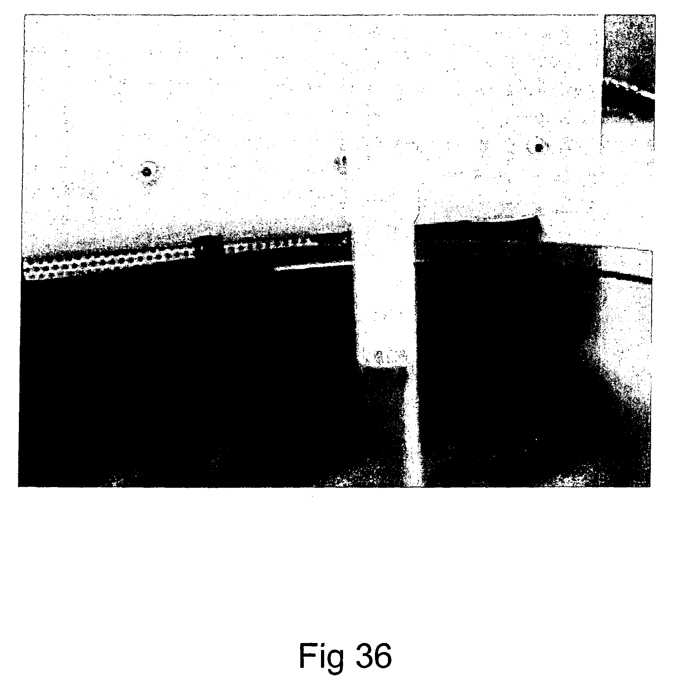

Figure 36 shows a disc of microwell-containing silicone rubber inserted into a standard plastic cryovial.

### Example 32: Solvent welding of 50 micron Nylon mesh onto 200 micron polystyrene microwells.

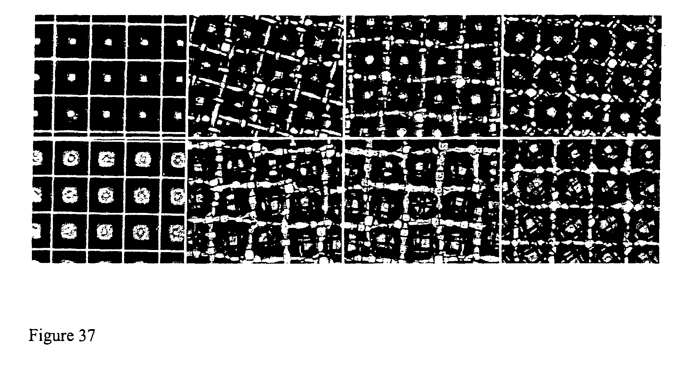

Figure 37 shows solvent welding of 50 micron Nylon mesh onto 200 micron polystyrene microwells. Upper row: imaged from below (looking through the polystyrene), lower row, imaged from above (looking through the mesh). Polystyrene microwells (left column) were juxtaposed to a Nylon mesh sized for 24-well plate, and ∼25 microliters of butanone-hexane solution were dispensed over the top. Dilutions used were: 1 volume butanone to 3 volumes hexane (2nd column from left), 1 volume butanone to 3.5 volumes hexane (3rd column from left), 1 volume butanone to 4 volumes hexane (4th column from left). Note that the effect on contact area and hence strength of adhesion may be titrated by altering the dilution ratios. Selection of solvent and diluent to be used depend on the precise materials from which the microwells and mesh are constituted, as well as the nature of the attachment (temporary or permanent) desired.

### References

1. Pera_2004: Martin F Pera and Jessica Andrade and Souheir Houssami and Benjamin Reubinoff and Alan Trounson and Edouard G Stanley and Dorien Ward-van Oostwaard and Christine Mummery, Regulation of human embryonic stem cell differentiation by BMP-2 and its antagonist noggin.; J Cell Sci 117(Pt 7): 1269-1280; 2004.
2. Ng_2005: Elizabeth S Ng and Richard P Davis and Lisa Azzola and Edouard G Stanley and Andrew G Elefanty, Forced aggregation of defined numbers of human embryonic stem cells into embryoid bodies fosters robust, reproducible hematopoietic differentiation.; Blood 106(5): 1601-1603; 2005.
3. Burridge_2007: Paul W Burridge and David Anderson and Helen Priddle and Maria D Barbadillo Muñoz and Sarah Chamberlain and Cinzia Allegrucci and Lorraine E Young and Chris Denning, Improved human embryonic stem cell embryoid body homogeneity and cardiomyocyte differentiation from a novel V-96 plate aggregation system highlights inter-line variability; Stem Cells 25:929--938 ; 2007.
4. Khademhosseini_2006: Ali Khademhosseini and Robert Langer and Jeffrey Borenstein and Joseph P Vacanti, Microscale technologies for tissue engineering and biology.;Proc Natl Acad Sci U S A 103(8): 2480-2487; 2006.
5. Mohr_2006: Jeffrey C Mohr and Juan J de Pablo and Sean P Palecek, 3-D microwell culture of human embryonic stem cells.; Biomaterials 27(36): 6032--6042; 2006.
6. Vallier_2004: ludovic vallier and daniel reynolds and roger a pedersen, nodal inhibits differentiation of human embryonic stem cells along the neuroectodermal default pathway.; dev biol 275(2): 403-421; 2004

## Claims

1. A microwell device comprising:
a) a body comprising an upper region defining an upper plane;
b) a plurality of microwells extending downwardly from the upper plane into the body;
c) a particle retaining member in communication with at least one of the microwells.

2. The microwell device of claim 1, further comprising a common fluid reservoir provided above the upper plane.

3. The microwell device of any of claims 1 or 2, wherein:
a) each of the microwells comprises an axis extending perpendicularly to the upper plane; and
b) each of the microwells comprising a sidewall, the sidewall of at least one of the microwells having at least one wall component extending inwardly towards the axis

4. The microwell device of any of claims 1 to 3, wherein the particle retaining member is a baffle configured to modulate a flow of fluid in the at least one microwell.

5. The microwell device of any of claims 1 to 3, wherein the particle retaining member is a filter member configured to trap particles of greater than a selected size within the at least one of the microwells.

6. The microwell device of claim 5, wherein the filter member is a porous sheet extending across the at least one of the microwells or each of the microwells.

7. The microwell device of any of claims 5 or 6, wherein the filter member seats on an upper region of the body, and/or is affixed to the upper region of the body, and/or is adhered to the upper region of the body.

8. The microwell deice of any of claims 5 to 7, wherein the filter member is removable from the upper region of the body.

9. The microwell device of any of claims 5 to 8, wherein the filter member comprises a lid covering only a portion of the at least one microwell.

10. The microwell device of any of claims 1 to 8, wherein the particle retaining member comprises a mesh.

11. The microwell device of claim 10, wherein the mesh comprises pores of between 37 microns and 75 microns in diameter.

12. The microwell device of any of claims 10 or 11, wherein the microwells are defined by a width at the upper plane, and the mesh comprises pores having a diameter of less than the width.

13. The microwell device of any of claims 1 to 12, wherein the particle retaining member is positioned below the upper plane within the at least one of the microwells, or above the upper plane in a common fluid reservoir.

14. The microwell device of any of claims 1 to 8, wherein the microwells are defined by a width at the upper plane, and the particle retaining member is spaced from the upper plane by a distance of less than the width, or wherein the particle retaining member comprises a mesh having pores having a diameter and the particle retaining member is spaced from the upper plane by a distance of less than the diameter, or wherein the particle retaining member is spaced from the upper plane by a distance less than one cell diameter.

15. The microwell device of claim 1, wherein the particle retaining member is configured to modulate a flow of fluid in the at least one of the microwells, and to trap particles of greater than a selected size within the at least one of the microwells.

16. The microwell device of claim 1, wherein the particle retaining member comprises a baffle and a filter member separate from the baffle.
